# EUROPEAN PATENT APPLICATION

(11) **EP 4 652 991 A1**
(43) Date of publication of application: **26.11.2025**
(21) Application number: 24382543.7
(22) Date of filing: 22.05.2024
(51) Int. Cl.: A61K 9/51, A61K 9/00, A61K 9/127, A61K 38/00, A61P 35/00, A61K 39/395

(54) **NANOASSEMBLIES FOR THE INTRACELLULAR DELIVERY OF ACTIVE PRINCIPLES OF AMINOACIDIC NATURE**

(71) Applicant: Universidade de Santiago de Compostela, 15782 Santiago de Compostela, La Coruña (ES)
(72) Inventor: LÓPEZ ESTÉVEZ, Ana María, 15782 Santiago de Compostela (A CORUÑA) (ES); TORRES LÓPEZ, María Dolores Ramona, 15782 Santiago de Compostela (A CORUÑA) (ES); ALONSO FERNÁNDEZ, María José, 15782 Santiago de Compostela (A CORUÑA) (ES)
(74) Representative: Balder IP Law, S.L.

(57) **Abstract**

The invention disclosed herein provides a delivery system for active molecules formed by nanoassemblies with a structure of a hybrid bilayer comprising an amphiphilic polymer, which may be functionalized with some moieties, and a phospholipid. This bilayer delimits an aqueous core in which an active molecule of aminoacidic nature has been incorporated. Furthermore, the nanoassemblies may incorporate a targeting moiety to direct the system to the target tissue. Moreover, the present invention also relates to a pharmaceutical composition comprising these nanoassemblies and to the therapeutic use of these nanoassemblies, as they are able to achieve an intracellular delivery and distribution of the active molecule into the target tissues.

## Description

### TECHNICAL FIELD

The invention disclosed herein provides a delivery system for active molecules formed by nanoassemblies with a structure of a hybrid bilayer comprising an amphiphilic polymer, which may be functionalized with some moieties, and a phospholipid. This bilayer delimits an aqueous core in which an active molecule of aminoacidic nature has been incorporated. Furthermore, the nanoassemblies may incorporate a targeting moiety to direct the system to the target tissue. These nanoassemblies are able to achieve an intracellular delivery and distribution of the active molecule successfully. Thus, the present invention belongs to the field of the nanostructures for drug delivery.

### STATE OF THE ART

Despite the great potential of biological drugs in general and antibodies, in particular, their full exploitation in therapies is being significantly constrained by a number of biopharmaceutical problems, including their susceptibility to degradation, their incapacity to cross biological barriers, their inadequate biodistribution or poor ability to reach the target tissues. In this context, nanotechnology, with particle sizes below 200 nm, preferably 100 nm, has emerged as a potential strategy to deal with the above-mentioned problems. Nevertheless, the design of the appropriate nanocarrier is not a simple and straight-forward approach. For example, a classical problem of nanodelivery carriers is related to their susceptibility to alteration upon contact with the blood stream, a process that impairs their targeting capacity and may exacerbate immune reactions against biological drugs. Although the use of materials such as PEG and other molecules that provide nanocarriers with stealth attributes has been part of the trials to provide a solution, significant variability in the outcomes has been highlighted as a major concern and limitation. The use of targeting ligands together with the rational design of the nanocarriers, based on the specific physiopathological characteristics of the target tissue, are expected to enhance the chances of the drugloaded carriers to reach their targets.

To overcome these problems, several strategies have been developed for the delivery of active principles of aminoacidic nature such as enzymes or monoclonal antibodies. For example, Ma and co-workers [ACS Nano. 13 (2019) 8890-8902)], developed nanoclustered enzymes consisting of covalently crosslinked glucose oxidase and catalase to induce apoptosis in tumor cells. These nanostructures were coated with a conjugate of BSA and the hypoxia-activated chemotherapeutic, tirapazamine. A different strategy to mitigate tumor hypoxia has been trying to enhance the efficacy of the treatments. To this aim, catalase was entrapped in nanocapsules or liposomes, in association with therapies, such as photodynamic [Biomaterials. 181 (2018) 310-317] or radio-immunotherapy [Cancer, Nano Lett. 18 (2018) 6360-6368], respectively. Similarly, different nano-delivery systems have been proposed for the intratumoral delivery of trastuzumab, which targets the HER2 [Nat. Nanotechnol. 9 (2014) 907-912] or bevacizumab (BVZ), [Drug Deliv. Transl. Res. 10 (2020) 635-645].

Document WO2019/086627A1 discloses nanoassemblies, such as nanocapsules, comprising an inner portion surrounded by an outer shell, the outer shell comprising polysialic acid (PSA), the PSA bonded to targeting moieties, particularly the cell penetrating peptides Lyp-1 or cLyp-1. They may also contain pharmaceutical agents, such as paclitaxel and docetaxel.

WO2015/171079A1 discloses micellar nanocomplexes for drug delivery, comprising a micelle and an agent encapsulated within said micelle, which comprises a polymerflavonoid conjugate, wherein said polymer is bonded to the B ring of the flavonoid. Among others, such polymer of the conjugate may be polyethylene glycol (PEG) and hyaluronic acid.

WO2020172263 discloses nanocapsules formed by a polymeric network, encapsulating a polypeptide cargo, e.g. an antibody, having a hydrolysable crosslinking moiety coupled to the polymers of the polymeric network. The nanocapsules may include a targeting agent coupled to the polymeric network.

In the context of therapies, the development of protein delivery strategies is still in early stages. Despite the number of nanomedicines that have made their way to the market, its clinical and preclinical failures observed are due to numerous factors yet to be resolved, such as NPs (nanoparticles) accumulation in liver and spleen, the limited diffusion across the tumor environment, the difficult access to metastatic niches and, ultimately, the hurdles associated to the intracellular delivery. The true potential of targeted delivery systems is yet to be realized, especially for the protein/peptide drugs.

### DESCRIPTION OF THE INVENTION

As an alternative to the technologies described above, the inventors have developed a novel delivery system, involving a combination of two key components: a modified amphiphilic polymer and a phospholipid, which form a hybrid bilayer. This bilayer delimitates an aqueous core, which contains active molecules of aminoacidic nature. The inventors have applied a technique that enables the structural organization of the components of the bilayer and the entrapment of a high loading of active molecules. Taking advantage of the presence of hydrophobic areas in the active molecules and their net positive charge in physiological conditions, it was possible to assemble the three components into reproduceable nanoassemblies, which could then optionally be functionalized with target moieties, to direct and optimize the therapeutic effect of the active molecules. Additionally, the nanoassemblies may additionally be PEGylated to provide a nanosystem, which could enhance, even more, the stealth properties and, eventually, further be functionalized with a targeting ligand.

A key characteristic of the nanoassemblies of the invention is their robustness and manufacturability, which gives them a long-term stability. The inventors have demonstrated that the nanoassemblies may be stored as a powder by performing freeze-drying studies. The nanoassemblies of the invention may be scalable, to obtain larger batches.

The biological assays have also demonstrated that the nanoassemblies reach the target tissue and release the active molecule. Some examples illustrate the development of nanoassemblies formed by C16-hyaluronic acid (HAC16) and phosphatidylcholine, which contain for example bevacizumab as active molecule. Said nanoassemblies present the ability to effectively associate with and retain the antibody during blood circulation. Furthermore, the HAC16-based nanoassemblies, incorporating tLyP1 peptide as a targeting moiety and mAb as active molecule, have shown efficacy in penetrating tumors, as well as to impair the proliferation and colony formation in KRAS-mutant lung cancer cell lines.

Thus, a first aspect of the present invention refers to nanoassemblies comprising an aqueous core and an outer shell surrounding said aqueous core, wherein:
a) the outer shell is a hybrid bilayer, the layers of said bilayer comprising: phospholipids and an amphiphilic polymer, or salts thereof, preferably sodium salt, wherein the phospholipids are selected from one or more phospholipids, each phospholipid comprising a phosphate group, which may be optionally modified with one of choline, ethanolamine or serine, and two C₄-C₂₈ hydrocarbon chains deriving from fatty acids, wherein the hydrocarbon chains may be the same or different one from another and may be saturated or unsaturated; and the amphiphilic polymer consists of repeating units of monomers consisting of carbon, oxygen, nitrogen and hydrogen atoms, which is substituted by linear C₂-C₂₄ alkyl groups with a substitution degree of between 1 and 100%, the substitution degree being defined as the average number of hydroxyl or amine groups replaced by said linear C₂-C₂₄ alkyl groups, the polymer having preferably a molecular weight ranging from 1 to 250 kDa;
b) the aqueous core comprises one or more active agents, selected from proteins, polypeptides and peptides;
and wherein the nanoassemblies have an average size of between 30 and 300 nm.

In the present invention, the term "nanoassemblies" refers to a nanometer-sized architecture, wherein at least one dimension is lower than 700 nm, preferably lower than 500 nm and more preferably between 30 and 300 nm. According to a preferred embodiment, the nanoassemblies are colloidal systems. According to a preferred embodiment, the nanoassemblies of the invention are substantially uniform with oval or spherical shape.

Specifically, the amphiphilic polymer is formed by monomers, selected from aminoacids, preferably arginine or glutamic acid, and saccharides, preferably glycosaminoglycans. More preferably, the amphiphilic polymer is selected from hyaluronic acid (HA), polyglutamic acid (PGA), the polyarginines hexaarginine (r6), octaarginine (r8) or dodecaarginine (r12), being the octaarginine (r8) the most preferred, or derivatives of them, such as salts or solvates.

The HA that may form the nanoassemblies of the present invention can present a wide range of molecular weights (Mw). Depending on the required Mw, the used HA may be a commercially available HA or a HA fragment obtained by methods known in the art, such as the use of a hyaluronidase enzyme. As non-limiting examples, HA, as used herein, also includes its conjugated base (hyaluronate). This conjugated base can be an alkaline salt of hyaluronic acid including inorganic salts such as, for example, sodium salt, potassium salt, calcium salt, ammonium salt, magnesium salt, aluminium salt and lithium salt, organic salts such as basic amino acid salts at neutral pH. According to a particular embodiment, the HA forming the nanoassemblies of the invention is a conjugated base, more particularly an alkaline salt, and even more particularly a sodium salt of hyaluronic acid. Combinations of multiple types of hyaluronic acid may also be used, e.g., as subunits of a hyaluronic acid chain, and/or as different molecules of hyaluronic acid. In some embodiments, some of the hyaluronic acid units are modified, for example, by attachment to polyethylene glycol, alkyl or other hydrophobic moieties, or the like. Hydrophobic moieties include hydrophobic molecules or portions thereof, e.g., an alkyl group, such as those discussed herein.

In a preferred embodiment, the amphiphilic polymer of the nanoassemblies of the invention is HA-C16, that is, hyaluronic acid, or a conjugated salt thereof, modified by C₁₆ alkyl groups, having a molecular weight between 30 and 250 kDa, preferably between 30 and 70 kDa, or between 150-250 kDa.

The polyglutamic acid (PGA) which may form the nanoassemblies of the present invention can present a variable degree of polymerization (i.e., the number of glutamic units in the PGA may be from 2 to 500), and a wide range of molecular weights (e.g. from 4 to 100 kDa). Combinations of different fragments of PGA, with variable numbers of glutamic units therein, are also possible. It should be noted that the glutamic units need not be identical, and can independently be the same or different, even within the same polyglutamic acid. As used herein, PGA includes, but is not limited to, its conjugated base (glutamate), and/or water-soluble salts of PGA, as e.g. the ammonium salt and metal salts of PGA, such as the lithium salt, sodium salt, potassium salt, magnesium salt, etc. In some embodiments, the PGA is present as the sodium salt of poly-L-glutamic acid, as poly-alpha-glutamic acid or as sodium salt of poly-a-glutamic acid. In some embodiments, one or more polyglutamic acid units are modified, for example, by attachment to polyethylene glycol, alkyl or other hydrophobic moieties, or the like. Hydrophobic moieties include hydrophobic molecules or portions thereof, e.g., an alkyl group, such as those discussed herein.

The hexaarginine or hexa-arginine (r6), octaarginine or octa-arginine (r8) and dodecaarginine or dodeca-arginine (r12) forming the nanoassemblies of the present invention is a polymer with a backbone formed by six, eight or twelve arginines respectively which may be modified with, for example, alkyl or other hydrophobic moieties, or the like. Hydrophobic moieties include hydrophobic molecules or portions thereof, e.g., an alkyl group, such as those discussed herein. As used herein, the hydrophobic portion are C₁₂ alkyl groups and may be covalently attached to the N-terminal end of the r6, r8 or r12. Residues within the r6, r8 or r12 can be all D-isomers, all L-isomers, or any suitable combination of D- and L-isomers. When no isomer is specified, it should be understood that any isomer may be independently present in each residue in various embodiments.

In some embodiments, the moiety incorporated into the structure of any of the amphiphilic polymers mentioned above, is a linear alkyl group, preferably selected between C₁₂, C₁₄ and C₁₆ alkyl groups.

In a preferred embodiment, the amphiphilic polymer is selected from hyaluronic acid or hyaluronate (HA), substituted with C₁₆ alkyl groups (HA-C16), with a degree of substitution between 1% and 30%, preferably between 1% and 15%; polyglutamic acid (PGA), substituted with C₁₄ alkyl groups (PGA-C14), with a degree of substitution between 1 and 20%, preferably between 3% and 5%; and octaarginine (r8), substituted with C₁₂ alkyl groups (C12-r8), with a degree of substitution between 1 and 100%. The "degree of substitution" as used herein refers to the percentage of the total available sites on the polymer chain that have been substituted or modified (per base unit or per monomeric unit of the mentioned amphiphilic polymers.

Specifically, the active agent(s) in the aqueous core may be selected from a monoclonal antibody (mAb), preferably bevacizumab or anti-KRASG12V mAb; a peptide hormone, preferably insulin; or a protein growth factor, preferably connective tissue growth factor.

Specifically, the phospholipid may be selected from phosphatidylcholine (PC), phosphadidylethanolamine (PE), phosphatidylserine (PS), egg phospholipids, soybean phospholipids, synthetic phospholipids, hydrogenated phospholipids, PEGylated phospholipids, lysophosphaditylcholine. In a preferred embodiment, the phospholipid is PC. Further, the PC forming the nanoassemblies of the present invention may have different origin, such as e.g. from soybean, egg yolk or from animal tissues.

Preferably, the mass ratio of amphiphilic polymer: phosphatidylcholine in the nanoassemblies may be between 1:0.01 and 1:10, more particularly between 1:0.05 and 1:4 or between 1:0.06 and 1:4.

In some embodiments, the outer shell comprises polyethylene glycol) (PEG). In some cases, the PEG may be conjugated to the amphiphilic polymer, e.g., to form a hyaluronic acid-polyethyleneglycol acid copolymer (HA-PEG). However, in other cases, PEG is present in the outer shell of the nanoassemblies according to the present invention, but not conjugated to the amphiphilic polymer, but to any other component present in the outer shell, such as e.g. the phospholipid or the targeting moiety.

Preferably, the outer shell further comprises a polyethylene glycol (PEG) derivative containing at least 80% of ethylene glycol, for example phosphatidylethanolamine conjugated with PEG chains (PEGylated phospholipid) or derivatives thereof, such as 1,2-distearoyl-sn-glycero-3-phosphoethanolamine-N-[methoxy(polyethylene glycol)-2000 (DSPE.PEG2000).

In some embodiments, the components of the nanoassemblies may be functionalized. The terms "functionalize" and "functionalization" as used herein, indicates the appropriate chemical modifications of a molecular structure (including a substrate or a compound) resulting in attachment of a functional group to the molecular structure.

In some embodiments, the amphiphilic polymer, and/or the PEGylated phospholipid, if present, may be further functionalized with a targeting moiety, which may be a peptide, preferably selected from a cell-penetrating peptide and/or a tumor/tissue-penetrating peptide. More preferably, the targeting moiety is selected from tLyP1, iRGD, and cyclic LyP-1 and angiopep, preferably angiopep-2.

The targeting moiety is used to target the delivery of the active molecule included in the nanoassemblies to certain cell populations of an affected tissue of a subject. For instance, the targeting moiety facilitates the access of the nanoassemblies to one particular type of cell, (e.g., a cancer or tumor cell, an endothelial cell, or an immune cell) or to a specific organ, to release the active molecule that they carry. In some cases, the nanoassemblies are internalized by the cells with no need for targeting moieties, and in some other cases, internalization is facilitated and/or enhanced by the targeting moiety.

Another aspect of the invention refers to a process for preparing the nanoassemblies defined above, the process comprising the steps of:
i) providing an aqueous solution of one or more active agents selected from a protein, polypeptide or peptide, preferably with a concentration ranging from 0.05 to 30 mg/mL, preferably from 0.06 to 24 mg/mL;
ii) providing a solution of amphiphilic polymer in water, optionally in ethanol/water, preferably the content of ethanol ranging from 0 to 50 % (v/v), and preferably the concentration of the amphiphilic polymer being between 0.5 and 4 mg/mL; the solution optionally further comprising a targeting moiety;
iii) providing a solution of phospholipid, preferably phosphatidylcholine, and optionally PEGylated phospholipid, preferably DSPE.PEG2000, in ethanol, preferably the concentration of phosphatidylcholine being from 1 to 80 mg/mL, preferably from 2.5 to 60 mg/mL, and preferably the concentration of PEGylated phospholipid being from 0.1 to 40 mg/mL, preferably from 0.4 to 10 mg/mL; wherein the PEGylated phospholipid optionally comprises a targeting moiety;
iv) mixing the solution obtained in step i) with the solution obtained in step ii), preferably in a volume ratio of between 1:1 and 1:16, more preferably between 1:2 and 1:8, and even more preferably 1:4;
v) adding the solution obtained in step iii), preferably in a volume ratio from 1:2 to 1:25, more preferably 1:3.125 to 1:12.5 with regard to the mixture obtained in step iv), thus obtaining the nanoassemblies.

Preferably, this process further comprises a step vi) for concentrating the obtained nanoassemblies. The concentration may be carried out by common techniques, such as e.g. tangential flow filtration (TFF) or nitrogen evaporation.

According to a particular embodiment, this process further comprises a step of freeze-drying of the obtained nanoassemblies.

Another aspect of the invention refers to a pharmaceutical composition which comprises the nanoassemblies as defined above, and at least one pharmaceutical acceptable excipient.

In general, pharmaceutically acceptable excipients suitable for use in the invention are well-known to those of ordinary skill in the art. As used herein, a "pharmaceutically acceptable excipient" refers to a non-toxic material that does not significantly interfere with the effectiveness of the biological activity of the active compound(s) to be administered, but is used as a formulation ingredient, for example, to stabilize or protect the active compound(s) within the composition before use.

Another aspect of the invention refers to the nanoassemblies as defined above for use as a medicament or in therapy. Preferably, the nanoassemblies of the invention may be used for the treatment of cancer, as they are able to deliver and release into the tumoral cells the active compound which they carry. The active compound may have a direct cytotoxic activity or be capable of inducing cytotoxic processes in the tumoral cells. More preferably, the active compound is an antibody, such as bevacizumab or AntiKRAS^{G12V} mAb.

Another aspect of the invention refers to a method of treatment of a disease, which comprises administering to a subject in need thereof a therapeutically effective amount of the nanoassemblies of the invention. Preferably, said disease is cancer.

A "therapeutically effective amount" as used herein means the amount necessary to delay the onset, inhibit the progression, halt altogether the onset or progression, of a diagnosed particular condition to be treated, or otherwise achieve a medically desirable result. The terms "treat," "treating," and the like, generally refer to administration of the inventive compositions to a living organism.

The definitions and explanations exposed above are provided to better define the present invention and to guide those of ordinary skill in the art in the practice of the present invention. Unless otherwise noted, terms are to be understood according to conventional usage by those of ordinary skill in the relevant art. Each aspect so defined may be combined with any other aspect or aspects, unless clearly indicated to the contrary. In particular, any feature indicated as being preferred or advantageous may be combined with any other feature or features indicated as being preferred or advantageous. The definitions mentioned herein, apply *mutatis mutandis* to all the aspects disclosed in the present specification.

### BRIEF DESCRIPTION OF THE DRAWINGS

To complete the description and in order to provide a better understanding of the invention, a set of drawings is provided. Said drawings form an integral part of the description and illustrate aspects and embodiments disclosed herein, which should not be interpreted as restricting the scope of the invention, but just as an example of how the invention can be carried out. The drawings comprise the following figures:

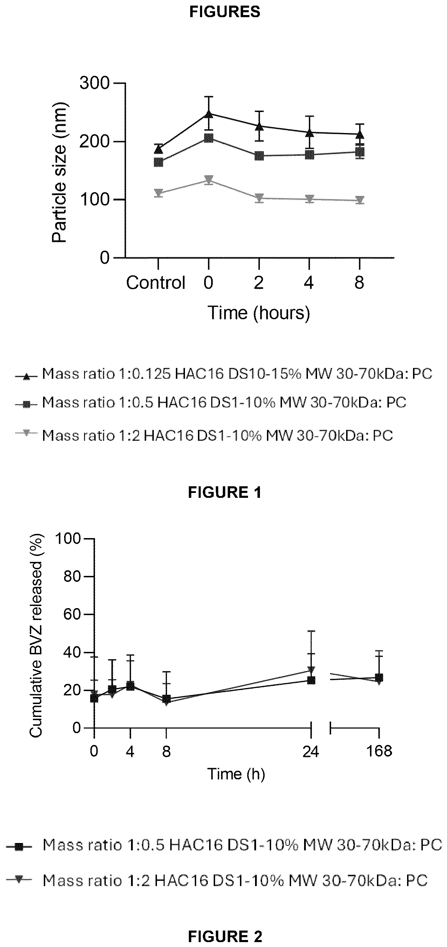

**Figure 1****.** Stability in simulated biological fluids at 37°C. NPs were incubated in PBS supplemented with 10% FBS and the distributive particle size was evaluated overtime, respectively. Control indicates the mean particle size of the NPs before incubation. Data are represented as mean ± SD, n=3. PBS phosphate buffer saline, FBS fetal bovine serum.
**Figure 2****.** Release profile of bevacizumab-loaded nanoassemblies upon incubation in PBS pH 7.4 at 37°C. The cumulative amount of released BVZ was quantified by ELISA. Data are represented as mean ± SD, n≥3.

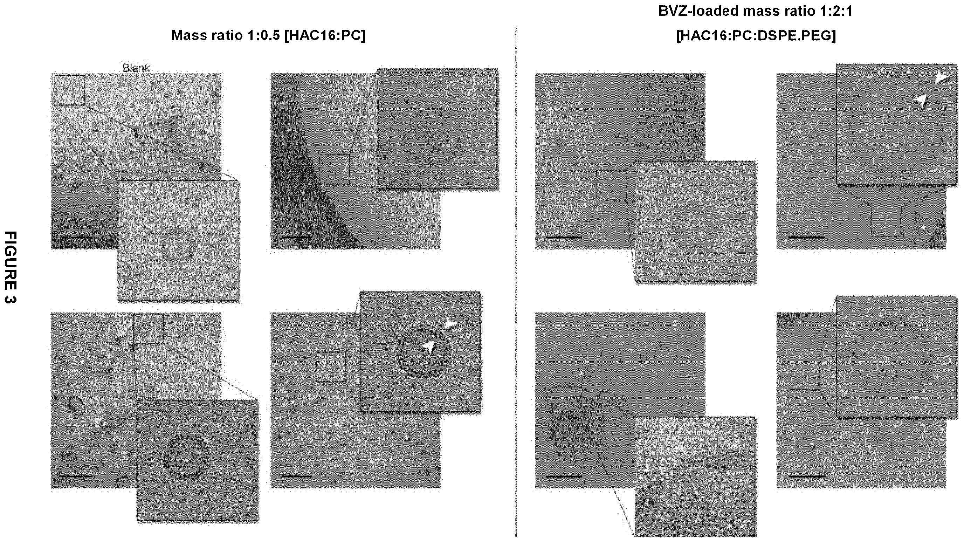

**Figure 3****.** Morphological characterization of Nanoassemblies. A gallery of cryo-TEM micrographs with a blank mass ratio 1:0.5 HAC16:PC, BVZ-loaded mass ratio 1:0.5 HAC16:PC, and BVZ-loaded mass ratio 1:2:1 HAC16:PC:DSPE.PEG; zoomed regions magnified 4X from the original size. The white arrows and asterisk mark the bilayer and irregular aggregates, respectively. The scale bars are 100 nm.

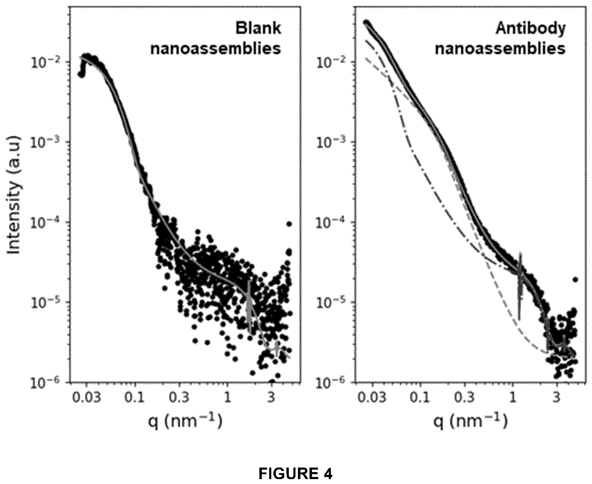

**Figure 4****.** SAXS profile of blank mass ratio 1:0.5 HAC16:PC sample (left) and BVZ-mass ratio 1:0.5 HAC16:PC sample (right).

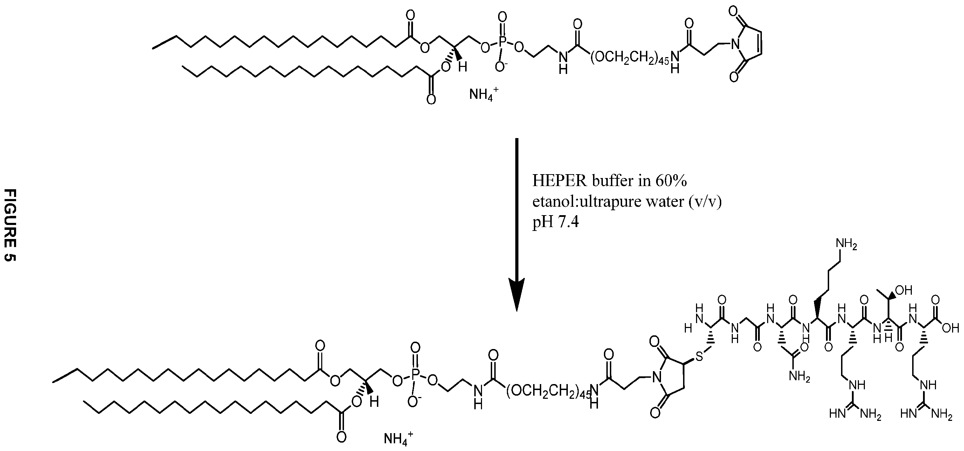

**Figure 5****.** Schematic representation of the reaction conditions for the obtention of the DSPE.PEGtLyP1 conjugate

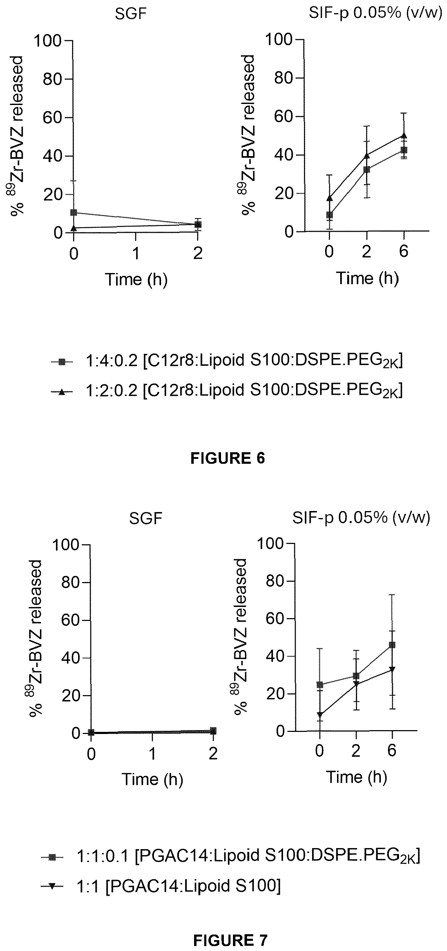

**Figure 6****.** BVZ release profile from C12r8-based nanoassemblies upon incubation in simulated gastric fluid (SGF) and simulated intestinal fluid supplemented with pancreatin (SIF-p 0.05%) at 37°C. Represented prototypes are reported in mass ratio at a final bevacizumab concentration of 0.5 mg/mL. The cumulative amount of released 89Zr-BVZ was quantified by iTLC. Data are represented as mean ± SD, n≥3.
**Figure 7****.** BVZ release profile from PGAC14-based nanoassemblies upon incubation in simulated gastric fluid (SGF) and simulated intestinal fluid supplemented with pancreatin (SIF-p 0.05%) at 37°C. Represented prototypes are reported in mass ratio at a final bevacizumab concentration of 1 mg/mL. The cumulative amount of released 89Zr-BVZ was quantified by iTLC. Data are represented as mean ± SD, n≥3.

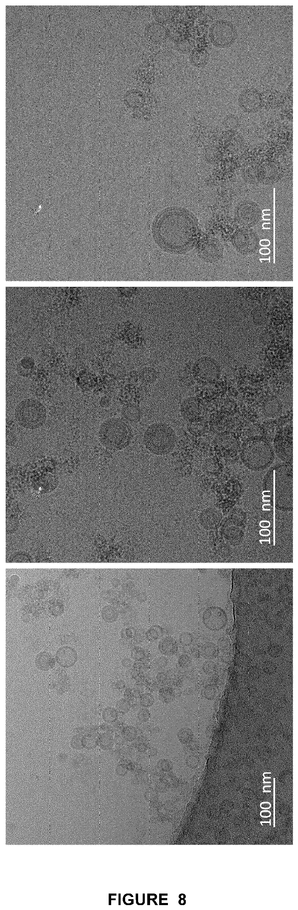

**Figure 8****.** Morphological characterization of PGA-based nanoassemblies loaded with bevacizumab (Mass ratio PGAC14:PC of 1:1 (w/w)). The scale bars are 100 nm.

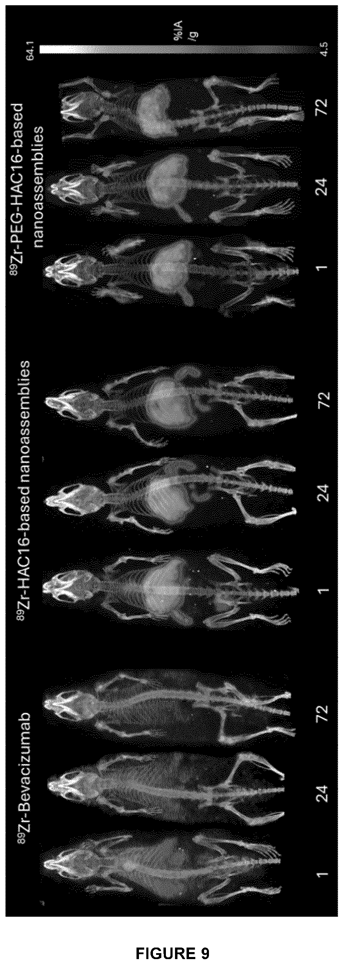

**Figure 9****.** Maximum intensity projection PET/CT imaging of 89Zr-bevacizumab, 89Zr-labeled bevacizumab-loaded HAC16-based nanoassemblies and 89Zr-labeled bevacizumab-loaded PEGylated HAC16-based nanoassemblies in healthy C57BL/6J mice. PET/CT maximum intensity projections after intravenous administration (n=4).

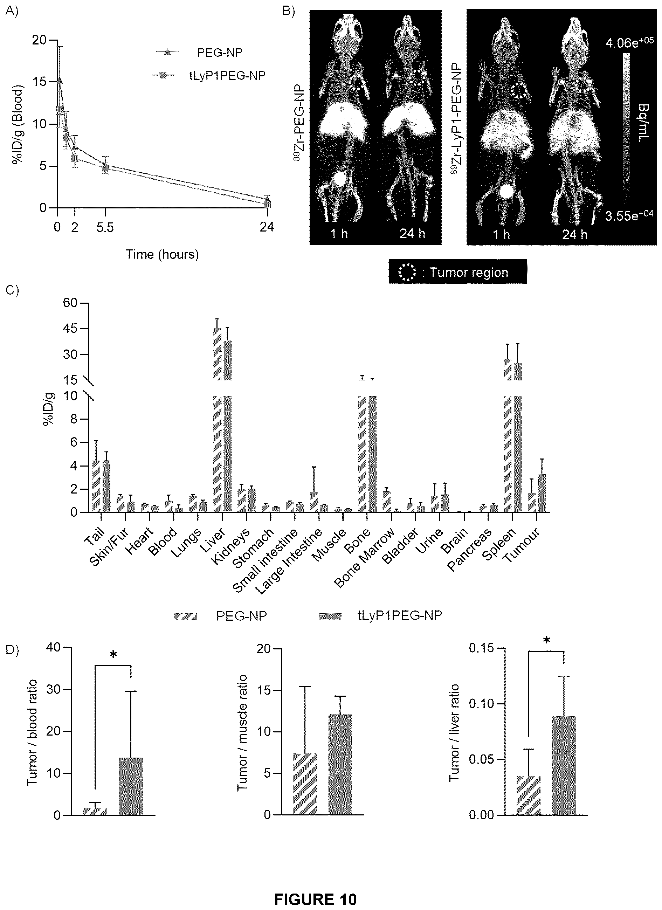

**Figure 10****.** Blood profiles, organ biodistribution, and PET imaging of 89Zr-labeled BVZ-loaded PEG-nanoassemblies (89Zr-PEG-NP) and 89Zr-labeled BVZ-loaded PEG-tLyP1-nanoassemblies (89Zr-PEG-tLyP1-NP) in an A549 tumor-bearing Balb/c mouse model. A) Percentage of the injected dose of 89Zr per gram (%ID/g) of blood after i.v injection. B) PET/CT maximum intensity projections of 2.5-3 (89Zr-PEG- NP) and 1.5-2.5 (89Zr-PEG-tLyP1-NP) after i.v. administration. C) Organ uptake represented as %ID/g of tissue at 24 h after i.v injection. D) Ratio of tumor/blood, tumor/muscle, and tumor/liver uptake. Data are represented as mean ± SD, n=4. Statistical comparison was done following an unpaired t-test (A, D tumor/muscle and tumor/liver ratio) and Mann-Whitney test (B, D tumor/blood ratio).

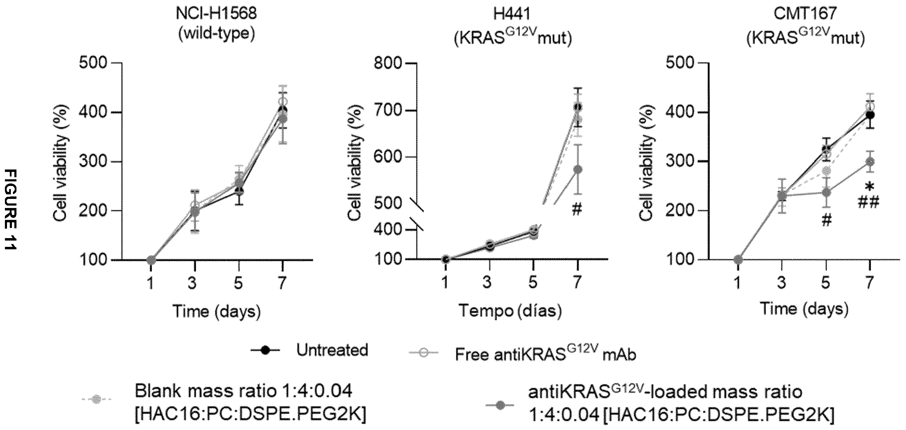

**Figure 11****.** AntiKRASG12V mAb-loaded PEGtLyP1-nanoassemblies impaired G12V mutated lung cancer cell proliferation. Lung cancer cell line was incubated for 7 days with culture medium (untreated), free antiKRASG12V mAb, blank, and antiKRASG12V mAb PEGtLyP1- nanoassemblies. Data are expressed as mean ± SEM, n≥3. Statistical differences were analyzed by multiple t-test at each time point between groups. # and * refers to differences among the free antiKRASG12V mAb or blank PEGtLyP1-nanoassemblies, and antiKRASG12V mAb PEGtLyP1-nanoassemblies, respectively. Differences among the antiKRASG12V mAb PEGtLyP1-nanoassemblies and the untreated group were not plotted.

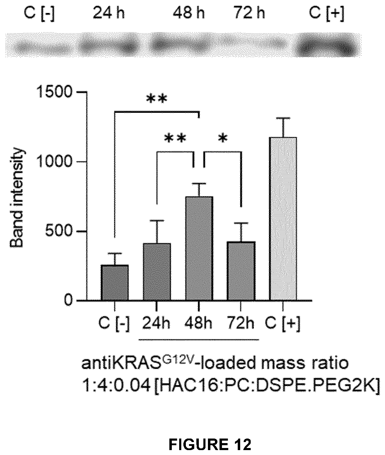

**Figure 12****.** Results of the target engagement study consisting of the analysis by immunoprecipitation (IP) of the interaction of KRASG12V protein with the antiKRASG12V mAb delivered from the PEGtLyP1-Nanoassemblies. The amount of IP product was determined at 24, 48, and 72 hours in the CMT167 cell line. The negative control [Ct (-)] was done by immunoprecipitating 1 mg of the CMT167 cell lysate with the anti-mAb beads. For the positive control [Ct (+)], 1 mg of the CMT167 cell lysate was added to 5 µg of the antiKRASG12V mAb. Statistical differences were determined by using a one-way ANOVA between groups but Ct (+). Data are represented as mean ± SD, n=3.

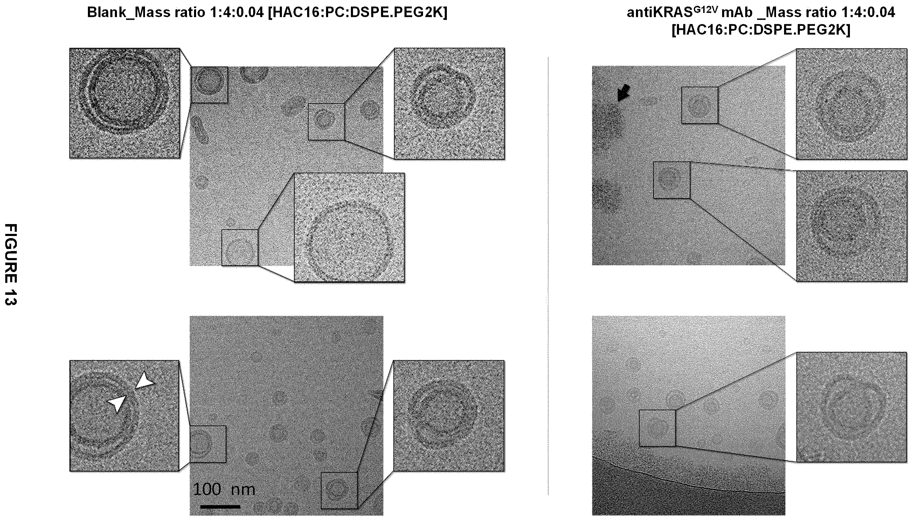

**Figure 13****.** In vivo efficacy studies in the αKRASG12V-mutant subcutaneous pancreatic cancer model. Relative tumor volume over 18 days. Pancreatic tumor-bearing mice were intravenously injected with the nanoassemblies at antiKRASG12V mAb dose of 10 mg/kg, and the respective amount of blank nanoassemblies. As control, PBS was administered. Data represents mean ± SEM of 6-7 replicates. Statistical differences were determined by following a 2-way ANOVA following by a Tukey test. Significant differences between αKRAS^{G12V}-HANAs and PBS were considered for ***p<0.001, and ****p<0.0001, and αKRAS^{G12V}-HANAs and blank HANAs were considered for ##<0.01.

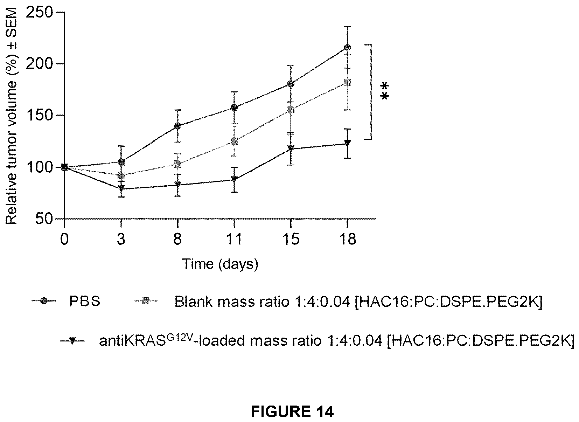

**Figure 14****.** Cryo-TEM micrographs of blank (top images) and antiKRASG12V mAb-loaded nanoassemblies (bottom images). Scale bar 100 nm, inset x3 enlarged from original; white arrowheads mark the ~3.5 nm thickness of the bilayer, the black-arrow presence of nanoaggregates.

### EXAMPLES

Following are examples of the invention by means of assays carried out by the inventors, which evidence the effectiveness of the product of the invention. The following examples serve to illustrate the invention and must not be considered to limit the scope thereof.

### MATERIALS AND METHODS

As an example, 1-step assembling method is used to produce the nanoassemblies. In general, this includes the preparation of an hydroalcoholic or aqueous solution that comprises an amphiphilic polymer (e.g., C12r8, PGAC14, HAC16) and protein, polypeptide or peptide (e.g., bevacizumab, anti-KRAS mAb, insulin or connective tissue growth factor) and an organic phase. The organic phase comprises one or more phospholipids. Nanoassemblies are obtained after mixing the solutions together. The organic solvents are completely or partially evaporated or removed.

As another option, a microfluidics method is used. For instance, the method includes the preparation of the hydroalcoholic or aqueous solution that comprises an amphiphilic polymer and the antibody, and an organic phase. The hydroalcoholic or aqueous solution could be prepared by microfluidics or not. The organic phase comprises at least one phospholipid (i.e., phosphatidylcholine). Nanoassemblies are obtained after mixing the solutions together.

In other examples the method of producing the nanoassemblies comprises an additional step of lyophilization, which may preserve them during storage. In all the tested cases, the addition of the cryoprotectant trehalose is needed to preserve them. In lyophilized form, nanoassemblies can be regenerated by adding water.

### Example 1.

This example illustrates the development of C16-hyaluronic acid (HAC16) nanoassemblies for the association of bevacizumab utilizing the amphiphilic polymers with two degrees of substitution of the C16 chains [DS 1-10% or C10-15%].

HAC16 nanoassemblies were prepared as follows. HAC16 was dissolved in a 6:4 (v/v) in ultrapure water:ethanol and phosphatidylcholine (PC) was prepared in ethanol. Over 500 µL of HAC16, 125 µL of bevacizumab were added under magnetic stirring in a fast one-step addition method. Then, 50µL of the PC solution were added drop-by-drop over the bevacizumab/HAC16 mixture. Thereafter, the volume was made up to 1mL with PBS. Table 1 and 2 summarize the final composition, mass ratio, and physicochemical properties of the tested conditions as mean ± SD of at least 3 replicates.

Table 3 summarizes the mass ratio and physicochemical properties after concentrate of selected candidates up to 3.2 mg/mL of bevacizumab as mean ± SD of at least 3 replicates. Remarkable high association efficiency and loading capacity was yielded (Table 3). Nanoassemblies presented high stability in simulated biological fluid (i.e., PBS supplemented with 10%FBS) at 37°C (Figure 1). The amount of released antibody after 1 week of incubation was lower than 30%, demonstrating the capacity of the nanoassemblies to retain the cargo for long periods (Figure 2). Additionally, a freeze-drying study was performed to assess the possibility to process mAb-containing nanoassemblies suspensions as powders for long-term storage. Results corresponding to at least 3 replicates are shown in Table 4, where it is shown that no significant changes were produced in terms of physico-chemical properties and AE.

**Physicochemical characterization.** Particle size, polydispersity index (PDI) and Zeta potential were measured by Dynamic Light Scattering (DLS) and laser Doppler anemometry (LDA) using a Zetasizer Nano-ZS (Malvern Instruments, Worcestershire, United Kingdom) with an angle detection of 173°. Samples were diluted in water or PBS accordingly.

**Isolation/concentration of bevacizumab-loaded nanoassemblies by a Tangential Flow Filtration (TFF) system or nitrogen source.** In the first approach, the nanoassemblies were submitted to a KrosFlo^{®} KR2i Tangential Flow Filtration (TFF) System (Repligen, Waltham, MA) with a membrane MWCO 100kDa (modified polyethersulfone, surface: 20cm2) run by a peristaltic pump. The nanoassemblies are added to the feed reservoir and will circulate through the TFF column in manual mode at an average flow rate 12 mL/min. After 5 diafiltrations, the instrument must be stopped when the volume in the feed reservoir corresponds to 3.2 mg/mL of bevacizumab. In the second approach, nanoassemblies are concentrated, removing the ethanol under a nitrogen stream.

**Bevacizumab association efficiency (AE, %) and loading capacity (LC, %).** AE was quantified by Enzyme-Linked ImmunoSorbent Assay (ELISA). Therefore, the nanoassemblies were isolated by ultracentrifugation at 35000 rpm, 1.5 h and 15°C. Two phases were formed: supernatant and pellet. The pellet was discarded, and the free antibody found in the supernatant was used for ELISA quantification. A 96 multiwell plate was coated with 0.005µg antigen/well (i.e. recombinant Human VEGF165) at a concentration of 0.05 µg/mL (100 µL/well). The antigen was prepared in coating buffer (Na₂CO₃ 1,59 mg/ml + NaHCO3 2,94 mg/ml (pH 9,59) + 0,5 mg/ml NaN3) and the coating of the plate was done overnight at 4°C. After the antigen attachment, the plate was washed four times with 300 µL/well of washing buffer (0.05% (v/v) Tween-20 in PBS, pH 7.4, 5 minutes per wash). The blocking was then performed with 300 µL/well of blocking buffer (2% (w/v) dry milk powder prepared in Washing Buffer) for 2h at 37°C. A further washing step is done and the antigen-coated plate is ready to use. The obtained free antibody in the supernatant were then loaded on the previously prepared antigen-coated plate and incubated for 1h at 37°C. Then, the plate was washed again with washing buffer and the secondary HRP-goat anti-human antibody added (0.08 µg/mL, prepared in coating buffer) and incubated for 1 hour at 37°C. The plate was washed again, and the detection substrate added to each well (50 µL/well). The association efficiency (AE, %) was calculated as [3.2- (mass of free bevacizumab)/(total theoretical mass of bevacizumab)] and the loading capacity (LC, %) as the mass of associated bevacizumab / (total theoretical mass of the antibody-loaded nanoassemblies)
**Release profile of the antibody.** NPs were diluted in PBS (1:10 dilution) and incubated at 37°C under orbital agitation. For each time point, an independent Eppendorf was prepared. At different time points, the sample was removed, isolated and the free antibody was quantified following the method described for the AE. Cumulative % of released bevacizumab was calculated as the cumulative amount of free antibody at each time point after subtracting the amount of free antibody before incubation.

**Freeze-drying studies.** The possibility of storage the samples as a powder was studied by performing freeze-drying studies. As non-limiting example, different bevacizumab-loaded HAC16-based nanoassemblies were prepared by the method explained above, and a concentrated solution of trehalose was added to the nanoassemblies suspension (final concentration of trehalose 2.5% w/v) prior to freeze-drying. Afterwards, the nanoassemblies were frozen at -80 °C and freeze-dried (Genesis^{™} 25 EL, S.P Industries, PA, USA). The freeze dried nanoassemblies were resuspended to their original volume in ultrapure water, and the particle size, PDI, Zeta potential, and AE were determined, and compared with the initial values (before freeze-drying).

**Table 1. Composition, mass ratio and physicochemical characterization of bevacizumab loaded nanoassemblies. Polymer used HAC16 30-70kDa DS 1-10%. Data are expressed as mean ± SD, n≥3.**

| **Final concentration (mg/mL)** | | | **Mass ratio BVZ:HAC16:PC (w/w)** | | | **Mass ratio HAC16:PC (w/w)** | | **Part. size (nm)** | **SD** | **PDI** | **SD** |
|---|---|---|---|---|---|---|---|---|---|---|---|
| **BVZ** | **HAC1 6** | **PC** | **BV Z** | **HAC1 6** | **PC** | **HA C16** | **PC** | | | | |
| 3 | 1 | 0,25 | 1 | 0,33 | 0,08 | 1 | 0,25 | 391 | 4 | 0,31 | 0,02 |
| 3 | 2 | 1 | 1 | 0,67 | 0,33 | 1 | 0,5 | 202 | 10 | 0,32 | 0,04 |
| 2 | 2 | 0,25 | 1 | 1 | 0,125 | 1 | 0,125 | 151 | 6 | 0,31 | 0,03 |
| 2 | 1 | 1 | 1 | 0,5 | 0,5 | 1 | 1 | 235 | 11 | 0,29 | 0,03 |
| 2 | 2 | 1 | 1 | 1 | 0,5 | 1 | 0,5 | 177 | 6 | 0,26 | 0,02 |
| 0,5 | 0,25 | 0,25 | 1 | 0,5 | 0,5 | 1 | 1 | 99 | 8 | 0,27 | 0,01 |
| 0,5 | 0,5 | 0,25 | 1 | 1 | 0,5 | 1 | 0,5 | 101 | 5 | 0,24 | 0,01 |
| 0,5 | 1 | 0,25 | 1 | 2 | 0,5 | 1 | 0,25 | 124 | 19 | 0,21 | 0,04 |
| 0,5 | 0,25 | 0,5 | 1 | 0,5 | 1 | 1 | 2 | 113 | 4 | 0,21 | 0,01 |
| 0,5 | 0,5 | 0,5 | 1 | 1 | 1 | 1 | 1 | 125 | 5 | 0,22 | 0,01 |
| 0,5 | 1 | 0,5 | 1 | 2 | 1 | 1 | 0,5 | 134 | 2 | 0,20 | 0,02 |
| 0,5 | 0,25 | 1 | 1 | 0,5 | 2 | 1 | 4 | 138 | 8 | 0,14 | 0,03 |
| 0,5 | 0,5 | 1 | 1 | 1 | 2 | 1 | 2 | 151 | 12 | 0,16 | 0,02 |
| 0,5 | 1 | 1 | 1 | 2 | 2 | 1 | 1 | 177 | 9 | 0,17 | 0,03 |

**Table 2. Composition, mass ratio and physicochemical characterization of bevacizumab loaded nanoassemblies. Polymer used HAC16 MW 30-70kDa DS 10-15%. Data are expressed as mean ± SD, n≥3.**

| **Final concentration (mg/mL)** | | | **Mass ratio BVZ:HAC16:PC (w/w)** | | | **Mass ratio HAC16:PC (w/w)** | | **Part. size (nm)** | **SD** | **PDI** | **SD** |
|---|---|---|---|---|---|---|---|---|---|---|---|
| **BVZ** | **HAC16** | **PC** | **BVZ** | **HAC16** | **PC** | **HAC16** | **PC** | | | | |
| 2 | 2 | 0,125 | 1 | 1 | 0,0625 | 1 | 0,0625 | 219 | 6 | 0.16 | 0.02 |
| 2 | 0,5 | 0,25 | 1 | 0,25 | 0,125 | 1 | 0,5 | 254 | 40 | 0.13 | 0.11 |
| 2 | 1 | 0,25 | 1 | 0,5 | 0,125 | 1 | 0,25 | 252 | 14 | 0.14 | 0.04 |
| 2 | 2 | 0,25 | 1 | 1 | 0,125 | 1 | 0,125 | 186 | 11 | 0.20 | 0.01 |
| 2 | 0,5 | 0,5 | 1 | 0,25 | 0,25 | 1 | 1 | 300 | 29 | 0.17 | 0.07 |
| 2 | 1 | 0,5 | 1 | 0,5 | 0,25 | 1 | 0,5 | 248 | 8 | 0.20 | 0.07 |
| 2 | 2 | 0,5 | 1 | 1 | 0,25 | 1 | 0,25 | 182 | 7 | 0.20 | 0.02 |
| 2 | 0,5 | 1 | 1 | 0,25 | 0,5 | 1 | 2 | 300 | 24 | 0.22 | 0.08 |
| 2 | 1 | 1 | 1 | 0,5 | 0,5 | 1 | 1 | 238 | 2 | 0.16 | 0.02 |
| 2 | 2 | 1 | 1 | 1 | 0,5 | 1 | 0,5 | 211 | 30 | 0.24 | 0.05 |

**Table 3. Physicochemical properties, association efficiency (AE, %), and loading capacity (LC, %) of the nanoassemblies at 3.2 mg/mL of bevacizumab. Data are expressed as mean ± SD, n≥3. Nanoassemblies were developed with the HAC16 DS 10-15% (^{a}) and DS 1-10% (^{b}) polymers.**

| **Mass ratio HAC16:PC (w/w)** | | **Physicochemical properties** | | | **AE (%)** | **LC (%)** |
|---|---|---|---|---|---|---|
| **HAC16** | **PC** | **Particle size (nm)** | **PDI** | **Zeta potential (mV)** | | |
| 1^{a} | 0.125 | 196 ± 13 | 0.24 | -14 ± 1 | 99 ± 0.3 | 47 ± 0.1 |
| 1^{b} | 0.5 | 162 ± 17 | 0.23 | -13 ± 2 | 86 ± 11 | 34 ± 4 |
| 1^{b} | 2 | 107 ± 14 | 0.23 | -13 ± 3 | 74 ± 11 | 30 ± 4 |

**Table 4. Physicochemical characterization and association efficiency (AE %) before and after freeze-drying studies in the presence of 2.5% (w/v) trehalose. Data are represented as mean ± SD, n≥ 3. Nanoassemblies were developed with the HAC16 DS 10-15% (^{a}) and DS 1-10% (^{b}) polymers.**

| **Mass ratio HAC16:PC (w/w)** | **Before freeze-drying** | | | | **After freeze-drying** | | | |
|---|---|---|---|---|---|---|---|---|
| | **Particle size (nm)** | **PDI** | **Zeta potential (mV)** | **AE (%)** | **Particle size (nm)** | **PDI** | **Zeta potential (mV)** | **AE (%)** |
| 1^{a}:0.125 | 196± 13 | 0.24 | -14 ± 1 | 99±0.3 | 192 ± 5 | 0.23 | -14 ±1 | 99± 0.5 |
| 1^{b}:0.5 | 162± 17 | 0.23 | -13 ± 2 | 86±11 | 167 ± 4 | 0.22 | -13 ±2 | 85 ± 3 |

### Example 2.

This example demonstrates the capability of nanoassemblies to form structures utilizing amphiphilic polymers with varying molecular weights. As a non- limiting example, HAC16 MW 150-250kDa DS 1-10% was used to formulate nanoassemblies with the mAb Bevacizumab.

The measurements of the particle size and PDI were done in the same way as previously described for mAb-loaded nanoassemblies (Example 1). Results are shown in Table 5.

**Table 5. Composition, mass ratio and physicochemical characterization of bevacizumab loaded nanoassemblies. Polymer used HAC16 150-250kDa DS 1-10%. Data are expressed as mean ± SD, n≥3.**

| **Final concentration (mg/mL)** | | | **Mass ratio BVZ:HAC16:PC (w/w)** | | | **Mass ratio HAC16:PC (w/w)** | | **Part. size (nm)** | **SD** | **PDI** | **SD** |
|---|---|---|---|---|---|---|---|---|---|---|---|
| **BVZ** | **HA** | **PC** | **BVZ** | **HA** | **PC** | **HA** | **PC** | | | | |
| 3 | 1 | 1 | 1 | 0,333 | 0,333 | 1 | 1 | 230 | 28 | 0,31 | 0,04 |
| 3 | 2 | 1 | 1 | 0,667 | 0,333 | 1 | 0,5 | 231 | 16 | 0,28 | 0,01 |
| 2 | 0.5 | 0.25 | 1 | 0,25 | 0,125 | 1 | 0,5 | 264 | 5 | 0,27 | 0,00 |
| 2 | 0.5 | 1 | 1 | 0,25 | 0,5 | 1 | 2 | 217 | 17 | 0,26 | 0,01 |
| 2 | 1 | 1 | 1 | 0,5 | 0,5 | 1 | 1 | 229 | 4 | 0,26 | 0,01 |
| 2 | 2 | 1 | 1 | 1 | 0,5 | 1 | 0,5 | 268 | 16 | 0,25 | 0,00 |

### Example 3.

This example demonstrates the capability to introduce a PEGylated lipid into the nanoassemblies. As a non- limiting example, DSPE.PEG2000 (Lipoid GmbH (Germany)) was used to formulate nanoassemblies with the mAb Bevacizumab.

PEGylated HAC16 nanoassemblies were prepared as follows. HAC16 was dissolved in a 6:4 (v/v) in ultrapure water:ethanol, PC and DSPE.PEG2000 were prepared in ethanol. Over 500µL of a HAC16 solution of 4 or 0.5 mg/mL, 125µL of a BVZ solution of 16 or 4 mg/mL added under magnetic stirring in a fast one-step addition method. Then, 50 µL of a PC were added drop-by-drop over the BVZ/HAC16 mixture. Thereafter, the volume was made up to 1mL with PBS. Afterwards, nanoassemblies were concentrated to the desired clinical dose. Table 1 summarized the mass ratio, particle size, PDI, Zeta potential, AE and LC after concentrate as mean ± SD of at least 3 replicates.

The measurements of the particle size, PDI, Zeta potential, AE and LC were done in the same way as previously described for mAb-loaded nanoassemblies (Example 1). Results are shown in Table 6.

**Table 6. Physicochemical properties, association efficiency (AE, %), and loading capacity (LC, %) of the PEGylated nanoassemblies at 3.2 mg/mL of bevacizumab. Data are expressed as mean ± SD, n≥3. NPs were developed with the HAC16 MW 30-70 kDa DS 1-10% polymer.**

| **Mass ratio HAC16:PC:DSPE.PEG2000 (w/w)** | | | **Physicochemical properties** | | | **AE (%)** | **LC (%)** |
|---|---|---|---|---|---|---|---|
| **HAC16** | **PC** | **DSPE.PEG2000** | **Particle size (nm)** | **PDI** | **Zeta potential (mV)** | | |
| 1 | 0.5 | 0.013 | 156 ± 11 | 0.26 | -13 ± 3 | 33 ± 18 | 13 ± 7 |
| 1 | 2 | 1 | 79 ± 8 | 0.25 | -13 ± 2 | 67 ± 21 | 22 ± 7 |

### Example 4.

This example illustrates the morphological and architectural organization of the C16-hyaluronic acid (HAC16) nanoassemblies for the association of bevacizumab.

The formulation procedure for the HAC16-based nanoassemblies was the same one than the described in the Example 1 for prototype of mass ratio 1:0.5 HAC16:PC and Example 3 for prototype of mass ratio 1:2:1 HAC16:PC:DSPE.PEG
Structural organization was assessed using 2D cryo-images, revealing the presence of unilamellar vesicles comprising a PC/polymer bilayer and an aqueous core, with consistent characteristics observed regardless of mAb entrapment into the nanoassemblies (Figure 3). The thickness of prototype of mass ratio 1:2:1 HAC16:PC:DSPE.PEG was estimated to range between 6-8 nm. Morphological analysis identified spherical, oval, and tubular-like shapes for prototype of mass ratio 1:0.5 HAC16:PC, while a predominant spherical shape was observed for prototype of mass ratio 1:2:1 HAC16:PC:DSPE.PEG. AF4 analysis revealed average Radius of gyration (Rg) values of Rn 45±1, Rw 53±3, and Rz 63±5. SAXS profiles, coupled with corresponding diameter distribution functions, reveal discernible 3D conformational disparities between empty (blank prototype of mass ratio 1:0.5 HAC16:PC) and bevacizumab-loaded nanoassemblies (mAb-loaded prototype of mass ratio 1:0.5 HAC16:PC) in solution (Figure 4). In this instance, employing a multi-concentric spherical-shell model facilitated curve fitting to characterize a bilayer shell with an approximate 4 nm thickness. This analysis yielded diameter sizes of 40 nm for blank prototype of mass ratio 1:0.5 HAC16:PC and 60 nm for mAb-loaded prototype of mass ratio 1:0.5 HAC16:PC, aligning with the AF4 measurements. Notably, SAXS profiles for both blank and mAb-loaded nanoassemblies exhibit broad similarities in curvature, further elucidating the structural properties of the nanoparticles.

**Physicochemical and morphological characterization by cryoTEM.** Cryo-TEM was conducted to evaluate the morphology and structure of the Nanoassemblies designated as prototype of mass ratio 1:0.5 HAC16:PC and prototype of mass ratio 1:2:1 HAC16:PC:DSPE.PEG. Prior to 2D imaging, a 3µL volume of the mass ratio 1:0.5 HAC16:PC sample was applied onto a Cu 300-mesh Quantifoil^{®} R2/2 grid. The sample was then incubated for 30 seconds before being vitrified using an EM GP2 automatic plunge freezer (Leica). Vitrification was achieved with a 1.5-second blot-time, carried out in conditions of over 90% humidity and approximately 8°C. Data acquisition was carried out on a JEM-2200FS/CR (JEOL, Ltd.) 200 kV TEM equipped with a K2 Summit direct detection camera (GATAN) at 25,000X or 30,000X for high magnification imaging. The mass ratio 1:0.5 HAC16:PC sample was vitrified using a Vitrobot Mark IV plunge freezer (Thermo Fisher Scientific), applying 4µL of diluted sample (2 units of prototype of mass ratio 1:2:1 HAC16:PC:DSPE.PEG per 1 unit of PBS) onto a Cu 300-mesh Quantifoil^{®} R2/1 grid with 20 seconds of incubation. The vitrification process involved a 3-second blot-time with the application of a -2-offset value. This was carried out under conditions of 70-80% humidity and at room temperature. 2D cryo-imaging of prototype of mass ratio 1:2:1 HAC16:PC:DSPE.PEG was performed as for the mass ratio 1:0.5 HAC16:PC sample.

**Physicochemical characterization by asymmetric flow field flow fractionation (AF4).** AF4 measurements were performed using an AF2000 separation system (Postnova Analytics, Landsberg, Germany) equipped with PN1130 isocratic pumps and degasser, an inline PVDF filter 0.1 µm (MilPCore, MA, USA) and an autosampler with an injection loop of 103 µL. The separation was performed on a channel utilizing an RC membrane with 1 kDa MWCO and a Teflon spacer with a thickness of 350 µm. The AF4 equipment was coupled online with a UV-Vis spectrophotometer (PN3211), a Multi-Angle Laser Light Scattering (MALS, PN3609), and a refractive index detector (RI, PN3150).

**Physicochemical and morphological characterization by small-angle x-ray scattering (SAXS).** SAXS measurements were performed in the BL11 NCD-SWEET beamline from the ALBA Synchrotron Light source in Barcelona, Spain. Samples were placed in a Kapton windows sample holder suitable for liquids. The incoming beam energy was set at 15 keV and 8 keV with a sample detector distance of 3600 mm. 2D patterns were recorded in a Pilatus 1M (Dectris, Switzerland) detector. Ninety patterns per sample were recorded during 1 second of exposure time, where no radiation damage effects were observed during measurements. Isotropic 2D patterns were integrated using the pyFAI library. Vesicles were modelized with a multi-shell spherical model to consider the bilayer shell.

### Example 5.

This example illustrates the possibility of increasing the batch production of nanoassemblies and establish a scalable technology. Thus, for example, larger batches of HAC16-based nanoassemblies were prepared at a 50X scale (50 mL-batch).

HAC16 nanoassemblies were prepared as follows. HAC16 was dissolved in a 6:4 (v/v) in ultrapure water:ethanol and PC S100 were prepared in ethanol. Over 25 mL of a solution of HAC16 at 4 mg/mL, 6.250 mL of an aqueous solution of bevacizumab at 16 mg/mL were added under mechanical stirring at 680 rpm (RW 20 DZM propeller stirrer, IKA Spain) in a fast one-step addition method. Then, 1.250 mL of a PC solution at 20 mg/mL were added drop-by-drop over the bevacizumab/HAC16 mixture. Thereafter, the volume was made up to 50 mL with PBS. Nanoassemblies were finally diafiltrated and concentrated up to 3.2 mg/mL BVZ by TFF.

The nanoassemblies were characterized in terms of particle size, PDI, Zeta potential and AE after concentration by TFF according to the methods described in Example 1. Results corresponding to 1 replicate are shown in Table 7.

**Table 7. Physicochemical properties, association efficiency (AE, %), and loading capacity (LC, %) of the scale-up compared to the small-size batch. Data are expressed as mean ± SD, n≥3, but 50 mL-batch, n=1. N.D. not determined.**

| **Prototype (batch)** | **Physicochemical properties** | | | **AE (%)** | **LC (%)** |
|---|---|---|---|---|---|
| | **Particle size (nm)** | **PDI** | **Zeta potential (mV)** | | |
| Small batch | 162 ± 17 | 0.23 | -12 ± 1 | 86 ± 11 | 34 ± 4 |
| 50 mL-batch | 157 ± 00 | 0.15 | -15 ± 0 | 64 ± 00 | 26 ± 0 |

### Example 6.

This example illustrates the development of C16-hyaluronic acid (C16HA) nanoassemblies for the entrapment of a connective tissue growth factor. HAC16 nanoassemblies were prepared as follows: HAC16 was dissolved in a 6:4 (v/v) in ultrapure water:ethanol and PC were prepared in ethanol. Over 250 µL of HAC16, 62.5 µL of connective tissue growth factor were added under magnetic stirring in a fast one-step addition method. Then, 25 µL of a PC solution were added drop-by-drop over the connective tissue growth factor/HAC16 mixture. Thereafter, the volume was made up to 0.5mL with PBS.

The nanoassemblies were characterized in terms of particle size, PDI, Zeta potential and AE, according to the methods described above in Example 1. Results corresponding the mean ± SD of at least 3 replicates are shown in Table 8.

The described nanoassemblies yielded a particle size close to 180 nm, low polydispersity and negative surface charge. High association efficiency and loading capacity was yielded.

**Table 8. Mass ratio, physicochemical characterization, association efficiency and loading capacity of HAC16-based nanoassemblies at a final connective tissue growth factor concentration of 8 µg/mL Data are expressed as mean ± SD, n≥3.**

| **Mass ratio HAC16:PC (w/w)** | | **Physicochemical properties** | | | **AE (%)** | **LC (%)** |
|---|---|---|---|---|---|---|
| **HAC16** | **PC** | **Particle size (nm)** | **PDI** | **Z-potential (mV)** | | |
| 1 | 0.5 | 184 ± 6 | 0.13 | -18 ± 3 | 87 ± 5 | 0.14 ± 0.01 |

### Example 7.

This example describes PEGylated HAC16 nanoassemblies functionalized or not with the tumor penetrating peptide tLyP-1.

The composition of the nanoassemblies is an outer shell comprise by a mixture of phospholipids (i.e., phosphatidylcholine and DSPE.PEG2000) and totally or partially the negatively charged HAC16 polymer. The DSPE.PEG2000is modified or not with the tLyP1 peptide. In the hydrophilic core, it could be found totally or partially the monoclonal antibody bevacizumab and/or HAC16. As a non-limiting example, the molecular weight of the polymer is used in this example is in the range of 30-70kDa and the degree of substitution of the C16 chains varies from 1-10%.

The functionalization of the DSPE.PEG2000was achieved by a covalent linkage between the PEG and the cysteine of the peptide (Michael addition type reaction). More in detail, DSPE.PEG2000 has a maleimide group that allows the reaction between the doble bond of the maleimide with the thiol group from the cysteine peptide. The number of tLyP-1 molecules per nanoparticle could be easily controlled by varying the amount of DSPE-PEG₂₀₀₀-tLyP1 conjugate added to the assembly. Thus, the degree of substitution and purity is controlled while the ability of the peptide to recognize the neuropilin-1 receptor, preserved.

DSPE-PEG₂₀₀₀-Maleimide-tLyP1 conjugate was synthesized following a maleimidethiol coupling reaction between the maleimide group of the PEG chains and the thiol group of the peptide. DSPE-PEG₂₀₀₀-Mal and tLyP-1 were dissolved at an initial concentration of 1mg/mL and 40mg/mL, respectively, in HEPES buffer 10mM (60% (v/v) ethanol in water, pH 7.2). Then, the peptide was placed into the DSPE.PEG2000solution. The molar ratio for tLyP1: DSPE-PEG2000-Mal was set at 2:1. After stirring at room temperature overnight, the unreacted peptide was removed by dialyzing against 150mM NaCl (2h) and then, two more cycles against water. Finally, the functionalized DSPE.PEG2000 was kept at -80°C for at least 1 h, freeze-dried, and stored at -20°C. The final product was analyzed by ¹H-NMR spectroscopy. Figure 5 summarizes the reaction conditions of the DSPE.PEG-tLyP1 conjugate.

HAC16 self-assemblies functionalized or not were prepared as follows. HAC16 was dissolved in a 6:4 (v/v) in ultrapure water:ethanol, PC and DSPE.PEG₂₀₀₀ were prepared in ethanol, and DSPE.PEG₂₀₀₀tLyP1 was dissolved in 90% ethanol/ultrapure water (v/v). Over 500 µL of a solution of HAC16 at 0.5 mg/mL, 125 µL of a solution of BVZ at 4 mg/mL were added under magnetic stirring in a fast one-step addition method. Then, 50 µL of PC: DSPE.PEG₂₀₀₀ solutions at 20 mg/mL and 10 mg/mL, respectively, were added drop-by-drop over the BVZ/HAC16 mixture. Thereafter, the volume was made up to 1 mL with PBS. For the tLyP-1 targeted nanoassemblies, a DSPE.PEG₂ₖtLyP1 solution at 0.4 mg/mL was used, and the above method was followed. Finally, nanoassemblies were concentrated by using a nitrogen evaporation method as described in Example 1. Table 9 summarized the final composition after concentrate and the particle size, PDI, Zeta Potential and AE as mean ± SD of at least 3 replicates. It was found that the tLyP-1 targeted NPs could incorporate an increasing number of tLyP-1 molecules. Described nanoassemblies yielded a particle size around 100nm, low polydispersity and negative surface charge. Moreover, both prototypes presented high stability in simulated biological fluid (i.e., PBS supplemented with 10%FBS) at 37°C. Remarkable high association efficiency and loading capacity was yielded.

**Table 9. Composition and physicochemical characterization, association efficiency, loading capacity and % of released antibody of targeted and non-targeted nanoassemblies after concentration. *tLyP-1 targeted material. Data are expressed as mean ± SD, n≥3.**

| **Mass ratio HAC16:PC:DSPE.PEG2K** (w/w) | | | **Physicochemical properties** | | | **AE (%)** | **LC (%)** |
|---|---|---|---|---|---|---|---|
| **HAC16** | **PC** | **DSPE. PEG2k** | **Particle size (nm)** | **PDI** | **Zeta potential (mV)** | | |
| 1 | 2 | 1 | 79 ± 8 | 0.25 | -13 ± 2 | 67 ± 21 | 22 ± 7 |
| 1 | 2 | 0.04* | 121 ± 14 | 0.25 | -13 ± 2 | 65 ± 12 | 26 ± 5 |

**Characterization of the DSPE.PEGtLyP1 conjugate.** ¹H-NMR spectra were recorded using a Bruker DRX-500 or NEO-750 spectrometer. DSPE.PEG₂₀₀₀-tLyP-1 was dissolved at 2mg/mL in DMSO-d6 and transferred into 5-mm NMR tubes. DSPE.PEG₂₀₀₀-Mal and tLyP-1 standard solutions at the same conditions were used as controls. The extent of the conjugation was calculated based on the integral peak corresponding to the methyl group of the threonine of the peptide at 1.05 ppm and the methyl groups of the DSPE at 0.85 ppm.

### Example 8.

This example describes HAC16-based nanoassemblies functionalized or not with the tumor penetrating peptide tLyP-1 through the hydrophilic moiety of the amphiphilic polymer.

The composition of the nanoassemblies is an outer shell comprise of phosphatidylcholine and totally or partially the negatively charged HAC16 polymer. The HAC16 amphiphilic polymer is modified or not with the tLyP1 peptide. In the hydrophilic core, it could be found totally or partially the monoclonal antibody bevacizumab and/or HAC16. As a non-limiting example, the molecular weight of the polymer is used in this example is in the range of 30-70kDa and the degree of substitution of the C16 chains varies from 1-10%.

The number of tLyP-1 molecules per nanoparticle could be easily controlled by varying the amount of HAC16-tLyP1 conjugate added to the assembly. Thus, the degree of substitution and purity is controlled while the ability of the peptide to recognize the neuropilin-1 receptor, preserved.

The functionalization process of the tLyP1 peptide the HAC16, with a molecular weight range of 30-70 kDa and a degree of substitution of 1-10%, was conducted through a two-step reaction. Prior to this, stock solutions were prepared as follows: HAC16 (4 mg/mL) in 40% (v/v) ethanol in ultrapure water, EDC (20.8 mg/mL), and Maleimide (267 mg/mL) in ultrapure water, while NHS (20.8 mg/mL) was dissolved in ethanol. In the first step, 100 µL of MES buffer pH 6 (adjusted with TEA) was added to 5 mL of HAC16, followed by the sequential addition of 71.9 µL EDC for 5 minutes at room temperature (RT), 86.4 µL NHS for 10 minutes at RT, and 7.4 µL Mal for 2 hours at 40°C under magnetic stirring. The resulting product was purified by dialysis against a solution of 150 mM NaCl in ethanol (50% v/v EtOH/ultrapure water). The HAC16-Mal conjugate obtained was then frozen at -80°C and subjected to freeze-drying (Genesis^{™} 25 EL, S.P Industries, PA, USA). In the second step, the tLyP1 peptide was coupled to the HAC16-Maleimide intermediate by mixing the polymer (4 mg/mL) with the peptide in the presence of TCEP under magnetic stirring at RT. The reaction was conducted in HEPES buffer (60% (v/v) EtOH in ultrapure water, pH 7.2) with a roughly determined tLyP1:Maleimide molar ratio of 1:1. The resulting conjugate was purified by dialysis against a solution of 150 mM NaCl in ethanol (60% v/v) and ultrapure water. Subsequently, the HAC16-tLyP1 conjugate was frozen at -80°C, freeze-dried, and stored at -20°C for further use. The final product was analyzed by ¹H-NMR spectroscopy.

HAC16 nanoassemblies were prepared as follows. A solution of HAC16 was dissolved in a 6:4 (v/v) in ultrapure water:ethanol at 4 mg/mL and a solution of PC at 20 mg/mL were prepared in ethanol. Over 500 µL of HAC16, a solution 125 µL of bevacizumab at 16 mg/mL were added under magnetic stirring in a fast one-step addition method. Then, 50µL of a PC solution were added drop-by-drop over the bevacizumab/HAC16 mixture. Thereafter, the volume was made up to 1mL with PBS. For the tLyP-1 targeted HAC16-based nanoassemblies, HAC16-tLyP1 was shifted by HAC16, and the above method was followed. Nanoassemblies were finally diafiltrated and concentrated up to 3.2 mg/mL BVZ by TFF as described in Example 1.

The nanoassemblies were characterized in terms of particle size, PDI, Zeta potential, AE, LC and release profile after concentration by TFF according to the methods described in Example 1. Results as mean ± SD of at least 3 replicates are shown in Table 10.

**Table 10. Mass ratio and physicochemical characterization, association efficiency, loading capacity and % of released antibody of targeted and non-targeted nanoassemblies at a bevacizumab concentration of 3.2 mg/mL. *tLyP-1 targeted material. Data are expressed as mean ± SD, n≥3.**

| **Mass ratio HAC16:PC (w/w)** | | **Physicochemical properties** | | | **AE (%)** | **LC (%)** | **% Cumulative released antibody (%) after 1 week** |
|---|---|---|---|---|---|---|---|
| **HA C16** | **PC** | **Particle size (nm)** | **PDI** | **Z-potential (mV)** | | | |
| 1 | 0.5 | 162 ± 17 | 0.23 | -13 ± 2 | 86 ± 11 | 34 ± 4 | 27 ± 11 |
| 1* | 0.5 | 166 ± 9 | 0.18 | -11 ± 6 | 26 ± 11 | 10 ± 5 | 20 ± 20 |

**Characterization of the HAC16-tLyP1 conjugate.** 1H-NMR spectra was recorded using a Bruker DRX-500 or NEO-750 spectrometer. HAC16-Maleimide intermediate and HAC16-tLyP1 were dissolved in CD3OD/D2O, phosphate buffer saline D2O/CD3OD (1/1, v/v) at pH 7.4 at 2 mg/mL in DMSO-d6 and transferred into 5-mm NMR tubes. The extent of the conjugation was calculated as the number of double bond of the maleimide or tLyP1 molecules per 100 carboxylic acid groups of HAC16.

### Example 9.

This example illustrates the formulation of nanoassemblies with alternative amphiphilic polymers such as C12-octarginine (C12r8) for the efficient association and delivery of monoclonal antibodies and polypeptides. As a non-limiting example, bevacizumab and insulin were included in this example.

C12r8 nanoassemblies were prepared as follows. C12r8 was dissolved in ultrapure water, PC and DSPE.PEG2000 were prepared in ethanol. Over 500 µL of a solution of C12r8, 125 µL of bevacizumab or insulin were added under magnetic stirring in a fast one-step addition method. Then, 50µL of a PC solution or PC : DSPE.PEG2000 solution were added drop-by-drop over the bevacizumab or insulin /C12r8 mixture. Thereafter, the volume was made up to 1mL with PBS.

Table 11 and 12 summarized the mass ratio and particle size, PDI, Zeta potential, AE, and LC as mean ± SD of at least 3 replicates. The described nanoassemblies yielded a particle size ranging from 50 to 100 nm, low polydispersity and positive to neutral surface charge. Remarkable high association efficiency and loading capacity was yielded (see Table 11 and 12). Finally, the amount of released antibody was minor in simulated gastric fluid for up to 2 hours and moderate in simulated intestinal fluid (Figure 6).

**Table 11. Mass ratio, physicochemical characterization, association efficiency and loading capacity of C12r8-based nanoassemblies at a final insulin concentration of 0.3 mg/mL. Data are expressed as mean ± SD, n≥1.**

| **Mass ratio C12r8:PC:DSPE.PEG_{2K} (w/w)** | | | **Physicochemical properties** | | | **AE (%)** | **LC (%)** |
|---|---|---|---|---|---|---|---|
| **C12r8** | **PC** | **DSPE. PEG_{2K}** | **Particle size (nm)** | **PDI** | **Z-potential (mV)** | | |
| 1 | 4 | - | 99 ± 15 | 0.24 | +7 | 76 | 2.9 |
| 1 | 4 | 0.2 | 99 ± 8 | 0.26 | +2 | 89 ± 19 | 3 ± 1 |

**Table 12. Mass ratio, physicochemical characterization, association efficiency and loading capacity of C12r8-based nanoassemblies at a final bevacizumab concentration of 0.5 mg/mL. Data are expressed as mean ± SD, n≥3.**

| **Mass ratio C12r8:PC:DSPE.PEG_{2K} (w/w)** | | | **Physicochemical properties** | | | **AE (%)** | **LC (%)** |
|---|---|---|---|---|---|---|---|
| **C12r8** | **PC** | **DSPE. PEG_{2K}** | **Particle size (nm)** | **PDI** | **Z-potential (mV)** | | |
| 1 | 4 | 0.2 | 63 ± 5 | 0.17 | +1 ± 2 | 58 ± 22 | 16 ± 6 |
| 1 | 2 | 0.2 | 51 ± 4 | 0.21 | +1 ± 1 | 69 ± 16 | 27 ± 6 |

**Physicochemical characterization.** Particle size, PDI and Zeta potential were measured by Dynamic Light Scattering (DLS) and laser Doppler anemometry (LDA) using a Zetasizer Nano-ZS (Malvern Instruments, Worcestershire, United Kingdom) with an angle detection of 173°. Samples were diluted in water or PBS.

**Isolation/concentration of the insulin-loaded nanoassemblies.** The nanoassemblies were submitted to a KrosFlo^{®} KR2i Tangential Flow Filtration (TFF) System (Repligen, Waltham, MA) with a membrane MWCO 300kDa (modified polyethersulfone, surface: 20cm²) run by a peristaltic pump. The nanoassemblies are added to the feed reservoir and will circulate through the TFF column in manual mode at an average flow rate 12 mL/min. After 3 diafiltrations, the instrument must be stopped when the volume in the feed reservoir corresponds to 0.3 mg/mL of insulin. The isolated insulin-loaded nanoassemblies in the feed reservoir and the waste were collected.

**Insulin association efficiency (AE) and loading capacity.** The insulin was analyzed upon separation of the insulin-loaded nanoassemblies from the free insulin followed by direct analysis. Accordingly, the encapsulated insulin was determined in an aliquot of isolated nanoassemblies and the total amount of insulin was estimated in an aliquot of non- isolated nanoassemblies. The quantification of the insulin was performed by HPLC using a reversed-phase isocratic method (Agilent model, 1100 series LC with diodearray detector set at 214 nm, Santa Clara, USA) with a C18 column (Superspher^{®} RP-18 endcapped) as the stationary phase. The mobile phase was eluted at 1 mL/min and consisted of a 44:56 v/v mixture of phase A (93:7 v/v phosphate buffer (0.1 M, pH 2.3)-acetonitrile) and phase B (43:57 v/v phosphate buffer-acetonitrile). An isocratic program 55% A and 45% B was used. The AE (%) refers to the amount of insulin in the nanoassemblies compared to the initial amount of insulin. The final insulin loading (LC% w/w) was calculated by dividing the amount of associated insulin (AE x total insulin in the formulation) by the total weight of the nanoassemblies.

**Radiolabeling of the mAb with 89Zr.** BVZ was conjugated with deferoxamine through DFO-thioisocyanate (DFO-NCS) and the amines in the mAb to form the thiourea. The resulting BVZ-DFO conjugate was used for the radiolabeling with 89Zr. The reaction was performed in the presence of 1M oxalic acid containing 89Zr, 2 M sodium carbonate pH 7-8, 0.5 M HEPES buffer pH 7.2 and BVZ-DFO. After 1 hour of incubation, the [89Zr]-bevacizumab was concentrated using 100 kDa centrifugal filters to its original volume and use for the preparation of the nanoassemblies. The resulting nanoparticles were used to determine the association efficiency, loading capacity and release profile.

**Isolation/concentration of the bevacizumab-loaded nanoassemblies.** The nanoassemblies were isolated by using a centrifugal device of 300 kDa (12000 g, 1.5 min, room temperature). The non-associated [89Zr]-BVZ in the filtrate and the isolated [89Zr]-NPs were collected.

**Bevacizumab association efficiency (AE) and loading capacity.** The bevacizumab was analyzed upon separation of the bevacizumab-loaded nanoassemblies from the free bevacizumab followed by direct analysis. The quantification of the bevacizumab was performed by radio-instant Thin Layer Chromatography (radio-iTLC) using a 20mM citric acid, 60 mM EDTA:ACN (9:1) solution as mobile phase and a silica gel impregnated chromatography paper (Agilent Technologies, Agilent, Santa Clara, CA, USA) as solid phase. The reaction mixture was spotted in the chromatography paper and the sample was eluted with the mobile phase. Afterward, the radioactivity was measured using a iTLC radioactivity detector system (RITA, Elysia-Raytest, Angleur, Belgium) to calculate the AE using Gina Star Software (RITA, Elysia-Raytest, Angleur, Belgium). The AE (%) refers to the amount of [89Zr]-bevacizumab in the nanoassemblies compared to the amount of free and associated [89Zr]-bevacizumab. The final [89Zr]-bevacizumab loading (LC%) was calculated by dividing the amount of associated [89Zr]-bevacizumab (AE x total bevacizumab in the formulation) by the total weight of the nanoassemblies.

**Bevacizumab release profile.** NPs were diluted in diluted 5-times in simulated gastric fluid pH 4.0 and simulated intestinal fluid supplemented with pancreatin 0.05 % (w/v) and incubated at 37°C under orbital shaking at 350 rpm. For each time point, an independent Eppendorf was prepared. At different time points, the sample was removed, isolated and the released [89Zr]-bevacizumab was quantified following the method described for the AE.

### Example 10.

This example illustrates the formulation of nanoassemblies with alternative amphiphilic polymers such as C14-polyglutamic acid (C14PGA) for the efficient association and delivery of monoclonal antibodies.

C14PGA nanoassemblies were prepared as follows. PGAC14 was dissolved in ultrapure water, PC and DSPE.PEG2000 were prepared in ethanol. Over 500 µL of a solution of PGAC14 at 1 mg/mL, 125 µL of a bevacizumab solution at 4 mg/mL were added under magnetic stirring in a fast one-step addition method. Then, 50 µL of a PC solution at 10 mg/mL or PC:DSPE.PEG2000 solution (at 20 mg/mL and 2 mg/mL, respectively) were added drop-by-drop over the bevacizumab/PGAC14 mixture. Thereafter, the volume was made up to 1 mL with PBS.

The nanoassemblies were characterized in terms of particle size, PDI, and Zeta potential after concentration by nitrogen according to the methods described above. The AE was determined by iTLC after isolation by a centrifugal device of 300 kDa according to the method previously described in Example 9. Results corresponding the mean ± SD of at least 3 replicates are shown in Table 13.

The described nanoassemblies yielded a particle size close to or below 50 nm, low polydispersity and negative surface charge. High association efficiency and loading capacity was yielded. Moreover, resulting bevacizumab-loaded prototypes presented high stability in simulated gastrointestinal fluids at 37°C. Finally, the amount of released antibody was minor in simulated gastric fluid for up to 2 hours and moderate in simulated intestinal fluid (Figure 7).

**Table 13. Mass ratio, physicochemical characterization, association efficiency and loading capacity of PGAC14-based nanoassemblies at a final bevacizumab concentration of 1 mg/mL. Data are expressed as mean ± SD, n≥3.**

| **Mass ratio PGAC14:PC:DSPE.PEG_{2K} (w/w)** | | | **Physicochemical properties** | | | **AE (%)** | **LC (%)** |
|---|---|---|---|---|---|---|---|
| **PGA C14** | **PC S100** | **DSPE. PEG_{2K}** | **Particle size (nm)** | **PDI** | **Z-potential (mV)** | | |
| 1 | 1 | - | 54 ± 7 | 0.24 | -19 ± 2 | 64 ± 19 | 21 ± 6 |
| 1 | 1 | 0.1 | 44 ± 4 | 0.24 | -10 ± 2 | 62 ± 10 | 20 ± 3 |

The morphological analysis of bevacizumab-loaded PGAC14-based nanoassemblies was carried out with cryo-TEM and are shown in Figure 8. These results confirm the presence of a hybrid bilayer composed by the amphiphilic polymer PGA and phosphatidylcholine, surrounding an aqueous core.

### Example 11.

This example demonstrates the ability of nanoassemblies to effectively associate with and retain the antibody during blood circulation.

Prototypes designated in Examples 1 and 2 as prototypes 2 and 5, respectively, were formulated following the described procedure and their biodistribution profile was studied (Figure 9). PET/CT imaging were made at King's College London and were conducted in healthy C57BL/6 mice for up to 72 hours. ⁹Zr-bevacizumab remained in blood for long periods while the ⁸⁹Zr-bevacizumab-loaded nanoassemblies accumulated in the liver and spleen. These results confirmed the capacity of the nanoassemblies to retain the antibody and modify its biodistribution profile, confirming certain embodiments of the present invention.

**In vivo studies.** In vivo imaging was conducted in healthy 8-week-old C57BL/6 mice. Animals were anesthetized with isoflurane (2-3% in oxygen) and the radiolabeled NPs (1.5 - 3 MBq) intravenously administrated 1 h before the PET acquisition (1:5 coincidence mode; 5 ns coincidence time window). PET was recorded for 30 min and then a semicircular CT scan was performed. Animal body temperature and respiratory rate were monitored during the whole process. PET/CT images were reconstructed using Tera-Tomo 3D reconstruction (400-600 keV energy window, 1:3 coincidence mode, 4 iterations, and subsets) at a voxel size of (0.4 × 0.4 × 0.4) mm3 and corrected for attenuation, scatter, and decay.

### Example 12.

This example showcases the incorporation of the tLyP1 peptide into HAC16-based nanoassemblies, highlighting their efficacy in penetrating tumors. As non-limiting example, prototypes described in Example 7 were used for this study.

PET/CT studies were made at King's College London and were conducted in a subcutaneous A549 NSCL carcinoma mouse model to evaluate the influence of a tumor-penetrating peptide on biodistribution (Figure 10). Pharmacokinetic analysis revealed no significant differences up to 24 hours nanoassemblies in blood circulation. Ex vivo biodistribution studies at 24 hours aligned with PET/CT images, showing higher uptake in liver and spleen for 89Zr-PEG-nanoassemblies. Within tumors, high tumor to liver and tumor to blood ratios were evident for 89Zr-PEG-tLyP1-nanoassemblies, indicating enhanced tumor accumulation.

**In vivo studies.** Tumors were induced 1 week prior to the imaging studies by subcutaneous injection of 2×10⁶ A549 lung cancer cells in Balb/c nu/nu mice. Tumor growth and animal welfare were regularly monitored after that.

### Example 13.

This example illustrates the capacity of the HAC16 nanoassemblies to entrap other monoclonal antibodies with intracellular target such as anti-KRASG12V mAb (αKRASG12V), and be further granted by a PEG corona, which can be further functionalized through the PEG and/or HA motif with targeting and/or tumor/tissue-penetrating ligands as, for example, tLyp-1.

The composition of the nanoassemblies is an outer shell comprise by one or more phospholipids (i.e., phosphatidylcholine and/or DSPE.PEG2000) and totally or partially the negatively charged HAC16 polymer. The PEGylated lipid is modified with the tLyP1 peptide. In the aqueous core, it could be found totally or partially the monoclonal antibody αKRAS^{G12V} and/or HAC16. As non-limiting example, the molecular weight of the polymer used is in the range of 30-70 kDa and the degree of substitution of the C16 chains varies from 1-10%.

tLyP-targeted αKRAS^{G12V}-loaded nanoassemblies were prepared as follows. Prior to it, DSPE.PEG2000-tLyP1 conjugate was synthetized as described in Example 7. Hence, HAC16 was dissolved in a 6:4 (v/v) in ultrapure water:ethanol, PC was prepared in ethanol and DSPE.PEG2000-tLyP1 was dissolved in 90% ethanol/ultrapure water (v/v). Over 500 µL of a HAC16 solution at 0.5 mg/mL, 125 µL of αKRAS^{G12V} solution at 4 mg/mL were added under magnetic stirring in a fast one-step addition method. Then, 50 µL of a PC: DSPE.PEG2000-tLyP1 solutions at 40 mg/mL and 0.4 mg/mL, respectively, were added drop-by-drop over the αKRAS^{G12V} /HAC16 mixture. Thereafter, the volume was made up to 1 mL with PBS. NPs were concentrated 6.4-times by using a nitrogen evaporation method.

Table 14 summarized the final composition after concentrate and the physicochemical properties as mean ± SD of at least 3 replicates. It was found that the tLyP-1 targeted NPs could incorporate an increasing number of tLyP-1 molecules. Described nanoassemblies yielded a particle size around 140 nm, low polydispersity and negative surface charge. Moreover, tLyP-1 targeted nanoassemblies y yielded high stability in simulated biological fluid (i.e., PBS supplemented with 10%FBS) at 37°C. Remarkable high association efficiency and loading capacity was revealed. Finally, absence of significant release after 1 week of incubation at physiological pH at 37°C was observed.

**Table 14. Mass ratio, physicochemical characterization, association efficiency, loading capacity and % of released antibody of targeted nanoassemblies at a αKRAS^{G12V} concentration of 3.2 mg/mL. *tLyP-1 targeted material. Data are expressed as mean ± SD, n≥3.**

| **Mass ratio HAC16:PC:DSPE.PEG2000 (w/w)** | | | **Physicochemical properties** | | | **AE (%)** | **LC (%)** | **% Cumulative released antibody (%) after 1 week** |
|---|---|---|---|---|---|---|---|---|
| **HA C16** | **PC** | **DSPE. PEG2000** | **Particle size (nm)** | **PDI** | **Z-potential (mV)** | | | |
| 1* | 4 | - | 141 ± 7 | 0.20 | -11 ± 2 | 66 ± 9 | 19 ± 3 | 8 ± 10 |
| 1 | 4 | 0.04* | 142 ± 11 | 0.23 | -11 ± 2 | 64 ± 6 | 18 ± 2 | 26 ± 14 |

In this example, the capacity of the nanoassemblies composed by a mass ratio of 1:4:0.04 [HAC16:PC:DSPE.PEG2000] to deliver the antibody intracellularly, interact with the intracellular oncogene and block its function have been proved *in vitro* by antiproliferative (Figure 11) and target engagement (Figure 12) studies. Although the extent of the inhibition differs between cells line, the antiproliferative efficacy of αKRAS^{G12V}-loaded nanoassemblies is obvious after 7 days of incubation. As expected, no efficacy was observed in the wild-type cell line (NCI-H1568). Furthermore, target engagement experiments confirmed the intracellular delivery of the antibody, driven by the nanoparticles and the effective interaction of the KRAS protein with the αKRAS^{G12V}. Additionally, target engagement results demonstrated a higher KRAS target recognition after 48 hours.

In this example, the efficacy of the nanoassemblies was evaluated in a pancreatic cancer mice model. Results in Figure 14 show that nanoassemblies composed by a mass ratio of 1:4:0.04 [HAC16:PC:DSPE.PEG2K] significantly reduced tumor volume at the endpoint.

**Physicochemical characterization.** Particle size, PDI and Zeta potential were measured by Dynamic Light Scattering (DLS) and laser Doppler anemometry (LDA) using a Zetasizer Nano-ZS (Malvern Instruments, Worcestershire, United Kingdom) with an angle detection of 173°. Samples were diluted in PBS accordingly. Cryo-TEM was carried out for a structural analysis (Figure 1).

**Bevacizumab association efficiency (AE, %) and loading capacity.** The amount of associated αKRAS^{G12V} was determined by SDS-PAGE for separation under reducing conditions. NPs were isolated by ultracentrifugation following the previously described conditions (i.e., 35000rpm - 1.5 h -15°C). Then, the amount of free αKRAS^{G12V} present in the supernatant was quantified. Treated samples were diluted in a volume ratio 1:1 with 2X Laemmli buffer and vortexed for several seconds. Then, samples were boiled at 99.5°C for 5 minutes. 20µL of the denatured samples were resolved using stacking and resolving polyacrylamide gels (4 and 12%, respectively) in the presence of 0.1% SDS in the running buffer. After 1 hour of SDS-PAGE running, the gel was fixed in 10% methanol and 7% acetic acid for 45 minutes. Finally, samples were stained using a SYPRO^{®} Ruby protein gel stain solution overnight. Prior to analysis, the gel was washed twice with ultrapure water. GelAnalyzer 19.1 software (www.gelanalyzer.com) by Istvan Lazar Jr., PhD and Istvan Lazar Sr., PhD, CScs was used to processed and analyze the samples.

**Release pattern.** Cumulative % of released αKRAS^{G12V} was quantified by SDS-PAGE. Samples were diluted in PBS (dilution 1:10) at pH 7.4 and incubated at 37°C under horizontal agitation (300rpm). At different time points, the formulation was isolated by ultracentrifugation as described in Example 1. The free αKRAS^{G12V} in the supernatant was recovered and quantified.

**Antiproliferative studies.** Cell proliferation studies were performed in the human cell lines NCI-H441 and NCI-H1568 and the murine cell line CMT 167. 5 × 10¹ or 2.5 × 10¹ or cells/well were seeded in a 24-well plate. DMEM supplemented with a penicillin/streptomycin solution and 5%FBS as medium. Cells were treated on days 1, 3 and 5 at 37°C with αKRAS^{G12V}-loaded nanoassemblies at a 50 nM of the antibody and the corresponding amount of blank nanoassemblies and free antibody. Alamar Blue staining assay was used to measure the viability at 1, 3 and 7 days. Thus, the medium was discarded and 400µL/well of resazurin were added. After 40 minutes of incubation at 37°C, fluorescent levels were determined at 544-590 nm (Synergy H4, BioTek, VT, USA). As control, cells incubated in culture medium supplemented with 5% FBS and considered 100% viability. Scale reads to a negative control (Triton X100-0.5%) for comparison.

**Target engagement studies** were performed in the lung murine CMT167 cell line at densities of 0.5 × 10⁵ cells/well in a 6-well plate. The culture media of selection was DMEM supplemented with a penicillin/streptomycin solution and 10% FBS. Cells were treated with nanoassemblies at 50 nM of mAb. After 24, 48, or 72 h, cells were washed twice with PBS, and centrifuged at 1500 rpm 5min. Cell lysates were centrifuged at 140000 rpm for 15 min at 4 °C to remove nuclei and cell debris. Afterward, the supernatant was collected, and protein content was quantified by the bicinchoninic acid assay. To pull down the mAb, 1 mg of total protein (carrying a theoretical amount of 20 µg of mAb) was immunoprecipitated for 1 h with 40 µL of Protein G PLUS-agarose beads at 4°C in head-to-tail rotation. For positive target engagement control, 1 mg of CMT 167 cell lysate was incubated for 4 h at 4 °C (head-to-tail rotation) with 5 µg of free antiKRAS^{G12V} mAb and then immunoprecipitated as previously mentioned. Samples were loaded in 12 %-acrylamide PAGE gels and transferred to a PVDF protein blotting membrane. Membranes were incubated with antiKRAS mAb produced in mice followed by an anti-mouse IgG DyLight^{™} 680 antibody produced in goat. Finally, membranes were scanned using the LI-COR Odyssey Scanner system, and band intensities were quantified with LI-COR Image Studio^{™} Lite Quantification Software (LI-COR Biosciences, Lincoln, NE, USA).

***In vivo* efficacy studies.** Female NMRI-Foxn1 nu/nu mice (6-8 weeks old) were purchased from Janvier Labs (Le Genest-Saint-Isle, France). 3×106 CFPAC-1 cells were subcutaneously injected in the right flank (200 µl of cell suspension in a 1:1 matrigel:PBS mixture). When tumors reached approximately 100 mm3 animals were allocated into the different experimental groups (6-8 animals per group) with equal tumor size distribution (average and variance): one group received antiKRASG12V-PEGtLyP1-HANAs at antiKRASG12V dose of 10 mg/kg, other group received the respective amount of blank-HANAs and a third group, used as control, was injected only with PBS. Treatments were administered intravenously twice a week . Tumor size was measured twice a week in two dimensions using a caliper, and the volume was expressed in mm3 using the formula Tumor Volume=width2×length × 0.5. Animals were observed daily for clinical signs of pain or discomfort and weighted weekly.

### Example 14.

This example showcases the morphological and architectural organization of nanoassemblies formed by C16-hyaluronic acid (HAC16) for the association of an antiKRAS antibody. As a non-limiting example, the surface composition of the hybrid bilayer was elucidated, confirming the presence of amphiphilic polymers and phosphatidylcholine.

The morphological analysis by cryo-TEM (Figure 14) indicates lack of differences between empty and antibody-loaded nanoassemblies. Therefore, the morphological shape is intrinsic to the nanoassemblies and not dependent of the presence of the antibody. Morphologically, the images revealed heterogeneous populations that falls into spherical to oval shape and particle sizes <100 nm. The electron density differences indicated the presence of unilamellar or multilamellar vesicular structures consisting of a PC/polymer bilayer and an aqueous core.

**Surface characterization by X-ray photoelectron spectroscopy (XPS)** demonstrates the presence of phosphatidylcholine and the amphiphilic polymer on the surface.

The presence of phosphatidylcholine on the surface of the nanoassemblies is confirmed through P2p signals. At the elemental composition level, phosphorus is observed in phospholipids and nanoassemblies (prepared by either of the two methods) at similar magnitudes, albeit in lower proportions in the polymer (control). XPS elemental composition analysis confirms phosphorus presence in expected proportions, likely attributable to phosphatidylcholine on the nanoassemblies' surface. The anticipated presence of phosphate (PO4)-3 groups in phosphatidylcholine, a phospholipid, is consistent. Elemental phosphorus is typically observed in databases with P2p or p2p3/2 lines, exhibiting binding energies between 129 and 130 eV, whereas phosphate (PO4)-3 groups are commonly found at binding energies of 132.9-133.2 eV or slightly higher. Notably, the binding energy of the P2p signal in free phosphatidylcholine (control) aligns with that in the nanoassemblies, affirming the presence of phosphate groups from the phospholipid on the nanoassemblies' surface.

The presence of HAC16 on the surface of the nanoassemblies is confirmed through N1s signals. The N1s signal in the PC presents a single peak (only one Chemical environment) centered at 402.45 eV (100% relative). In the N1s signal of the polymer, two distinct peaks are observed, indicating two chemical environments. The primary peak, centered at 399.8 eV (91.66% relative intensity), is predominant, while the secondary peak appears at a higher binding energy (BE) of 402.6 eV (8.34% relative intensity), similar to that observed in phospholipids. Analysis of the N1s signal in the nanoassemblies under dry nitrogen conditions reveals two peaks: one at 399.4 eV, less abundant compared to the polymer (21.72% relative intensity), and another peak at 402.39 eV (78.28% relative intensity). Similarly, under freeze-drying conditions.

**Table 15. XPS Binding Energies and Relative Percent of N1s species of the free polymer and PC, and the respective nanoassembly for example 13 composed by a HAC16:PC S100: DSPE.PEG_{2K}-tLyP1 mass ratio of 1:4:0.04.**

| **Phosphatidylcholine (PC)** | | | | |
|---|---|---|---|---|
| **Name** | **Peak BE** | **FWHM eV** | **Area (P) CPS.eV** | **Atomic %** |
| N1s Scan A | 402.45 | 1.37 | 1767.26 | 100 |

| **HAC16 DS 1-10% MW 30-70 kDa** | | | | |
|---|---|---|---|---|
| **Name** | **Peak BE** | **FWHM eV** | **Area (P) CPS.eV** | **Atomic %** |
| N1s A | 399.8 | 1.47 | 2578.05 | 91.66 |
| N1s B | 402.63 | 1.39 | 234.02 | 8.34 |

| **Blank nanoassembly (Nitrogen)** | | | | |
|---|---|---|---|---|
| **Name** | **Peak BE** | **FWHM eV** | **Area (P) CPS.eV** | **Atomic %** |
| N1s A | 399.81 | 1.77 | 398.67 | 21.72 |
| N1s B | 402.39 | 1.17 | 1433.84 | 78.28 |

| **Blank nanoassembly (Freeze-drying)** | | | | |
|---|---|---|---|---|
| **Name** | **Peak BE** | **FWHM eV** | **Area (P) CPS.eV** | **Atomic %** |
| N1s A | 399.4 | 2.49 | 788.71 | 38.93 |
| N1s B | 402.36 | 1.47 | 1234.68 | 61.07 |

**2D-cryogenic transmission electron microscopy (cryo-TEM).** 2D-cryogenic transmission electron microscopy (cryo-TEM) micrographs were collected on a 120 kV TEM equipped with an UltraScan 4000 SP cooled slow-scan CCD camera (GATAN) at x30,000 magnification. High quality data were collected on a JEM-2200FS/CR (JEOL, Ltd.) 200 kV TEM equipped with a K2 Summit direct detection camera (GATAN) at x30,000 magnification. To do so, sample preparation was performed using the Vitrobot Mark IV, applying 4µL of HANAs (at 1:1 concentration) on a copper mesh. Vitrification was done in 70-80% humidity at RT. For quality control data was acquired on a JEM-1230 (JEOL, Ltd.)

## Claims

1. Nanoassemblies comprising an aqueous core and an outer shell surrounding said aqueous core, wherein:
a) the outer shell is a hybrid bilayer, the layers of said bilayer comprising: phospholipids and an amphiphilic polymer, or salts thereof, preferably sodium salt, wherein the phospholipids are selected from one or more phospholipids, each phospholipid comprising a phosphate group, which may be optionally modified with one of choline, ethanolamine or serine, and two C₄-C₂₈ hydrocarbon chains deriving from fatty acids, wherein the hydrocarbon chains may be the same or different one from another and may be saturated or unsaturated; and the amphiphilic polymer consists of repeating units of monomers consisting of carbon, oxygen, nitrogen and hydrogen atoms, which is substituted by linear C₂-C₂₄ alkyl groups with a substitution degree of between 1 and 100%, the substitution degree being defined as the average number of hydroxyl or amine groups replaced by said linear C₂-C₂₄ alkyl groups, the polymer preferably having a molecular weight ranging from 1 to 250 kDa;
b) the aqueous core comprises one or more active agents, selected from proteins, polypeptides and peptides;
and wherein the nanoassemblies have an average size of between 30 and 300 nm.

2. Nanoassemblies according to claim 1, wherein the monomers of the amphiphilic polymer are selected from aminoacids, preferably arginine or glutamic acid, and saccharides, preferably glycosaminoglycans.

3. Nanoassemblies according to any one of claims 1 or 2, wherein the amphiphilic polymer is selected from hyaluronic acid or hyaluronate (HA), polyglutamic acid (PGA), and octaarginine (r8).

4. Nanoassemblies according to any one of claims 1 to 3, wherein the substituent linear alkyl group of the amphiphilic polymer is selected between C₁₂, C₁₄ and C₁₆ alkyl groups.

5. Nanoassemblies according to any one of claims 1 to 4, wherein the amphiphilic polymer is selected from hyaluronic acid or hyaluronate (HA), substituted with C₁₆ alkyl groups (HA-C16), with a degree of substitution between 1% and 30%, preferably between 1% and 15%; polyglutamic acid (PGA), substituted with C₁₄ alkyl groups (PGA-C14), with a degree of substitution between 1 and 20%, preferably between 3% and 5%; and octaarginine (r8), substituted with C₁₂ alkyl groups (C12-r8), with a degree of substitution between 1 and 100%.

6. Nanoassemblies according to any one of claims 1 to 5, wherein the active agent(s) in the aqueous core is selected from a monoclonal antibody (mAb), a peptide hormone, or a protein growth factor.

7. Nanoassemblies according to any one of claims 1 to 6, wherein the phospholipid is phosphatidylcholine, preferably with a mass ratio amphiphilic polymer: phosphatidylcholine in the nanoassemblies being of between 1:0.05 and 1:4.

8. Nanoassemblies according to any one of claims 1 to 7, wherein the outer shell further comprises a polyethylene glycol derivatives containing at least 80% of ethylene glycol, phosphatidylethanolamine conjugated to polyethylene glycol (PEG) chains (PEGylated phospholipid) or derivatives thereof, preferably 1,2-distearoyl-sn-glycero-3-phosphoethanolamine-N-[methoxy(polyethylene glycol)-2000 (DSPE.PEG2000).

9. Nanoassemblies according to any one of claims 1 to 8, wherein the amphiphilic polymer, and/or the PEGylated phospholipid, if present, is further functionalized with a targeting moiety, which preferably is a peptide, more preferably selected from a cell-penetrating peptide and/or a tumor/tissue-penetrating peptide.

10. Nanoassemblies according to claim 9, wherein the targeting moiety is selected from tLyP1, iRGD, cyclic LyP-1 and angiopep-2.

11. Nanoassemblies according to any one of claims 1 to 10, wherein the amphiphilic polymer is HAC16 having a molecular weight between 30 and 250 kDa, preferably between 30 and 70 kDa or between 150 and 250 kDa.

12. A process for preparing the nanoassemblies of claims 1 to 11, the process comprising the steps of:
i) providing an aqueous solution of one or more active agents selected from a protein, polypeptide or peptide, preferably with a concentration ranging from 0.05 to 30 mg/mL, preferably from 0.06 to 24 mg/mL;
ii) providing a solution of amphiphilic polymer in water, optionally in ethanol/water, preferably the content of ethanol ranging from 0 to 50 % (v/v), and preferably the concentration of the amphiphilic polymer being between 0.5 and 4 mg/mL; the solution optionally further comprising a targeting moiety;
iii) providing a solution of phospholipid, preferably phosphatidylcholine, and optionally PEGylated phospholipid, preferably DSPE.PEG2000, in ethanol, preferably the concentration of phosphatidylcholine being from 1 to 80 mg/mL, preferably from 2.5 to 60 mg/mL, and preferably the concentration of PEGylated phospholipid being from 0.1 to 40 mg/mL, preferably from 0.4 to 10 mg/mL; wherein the PEGylated phospholipid optionally comprises a targeting moiety;
iv) mixing the solution obtained in step i) with the solution obtained in step ii), preferably in a volume ratio of between 1:1 and 1:16, more preferably between 1:2 and 1:8, and even more preferably 1:4;
v) adding the solution obtained in step iii), preferably in a volume ratio from 1:2 to 1:25, more preferably 1:3.125 to 1:12.5 with regard to the mixture obtained in step iv), thus obtaining the nanoassemblies.

13. Process according to claim 14, further comprising a step vi) of concentration and/or a step of freeze-drying.

14. A pharmaceutical composition comprising the nanoassemblies of claims 1 to 11, and at least one pharmaceutical excipient.

15. Nanoassemblies of claims 1 to 11, for use as a medicament or in therapy.
